# EUROPEAN PATENT APPLICATION

(11) **EP 4 763 335 A1**
(43) Date of publication of application: **24.06.2026**
(21) Application number: 24222596.9
(22) Date of filing: 20.12.2024
(51) Int. Cl.: B01L 3/00, B01L 9/00, C12M 1/00, C12M 1/12

(54) **SYSTEMS AND METHODS FOR TESTING BIOLOGICAL MATERIAL**

(71) Applicant: Koninklijke Philips N.V., 5656 AE Eindhoven (NL)
(72) Inventor: BECKERS, Lucas Johannes Anna Maria, Eindhoven (NL); VULDERS, Roland Cornelis Martinus, Eindhoven (NL); BAKKER, Frederikus, Eindhoven (NL); VALSTER, Susanne Maaike, Eindhoven (NL); KRIEGE, Jan Cornelis, Eindhoven (NL)
(74) Representative: Philips Intellectual Property & Standards

(57) **Abstract**

Provided is a system (100) for use in testing biological material. The system includes a substrate (102), and at least one support member (104) that has a biocompatible support portion (106) for supporting the biological material. The at least one support member is mountable to, and detachable from, the substrate. The at least one support member is detachable from the substrate at least by manual manipulation by a user, for example by the user pulling the at least one support member from the substrate using tweezers. Additionally provided is the support member per se. Further provided is a method of testing biological material using the system, as well as use of such a system for testing biological material.

## Description

### FIELD OF THE INVENTION

The invention relates to devices and systems for testing biological material, e.g. for testing biological material in cell biology and/or oncology tests.

The invention also relates to the use of such device and system for testing biological material.

The invention further relates to a method of testing biological material.

The invention yet further relates to a support member comprising a biocompatible support portion for supporting biological material and to a substrate of the device or system where the substrate is configured such that the support member is mountable to, and detachable from the substrate

### BACKGROUND OF THE INVENTION

In vitro testing of mammalian cells or tissue is an important technique to obtain clinically important information of the mammalian material under investigation. For example, biopsied mammalian cell or tissue material may be subjected to such testing to determine anomalies or disease in such mammalian material or to expose diseased mammalian material to drugs, e.g. experimental drugs, to monitor the response of the diseased mammalian material to such exposure. This approach, for example, is frequently used in oncological procedures. This can provide important insights in how a disease in an individual can be effectively treated without having to expose the individual to a range of potentially effective drugs, which can be undesirable for a number of reasons, including drug toxicity.

A common approach to such in-vitro testing is to immobilize the mammalian material in a fluidic device, which is sometimes also referred to as an "organ-on-chip." In such an approach, the mammalian material is typically immobilized on a membrane separating two sub-chambers of the fluidic device. One sub-chamber is used to contain the mammalian material and the other sub-chamber is used to expose the mammalian material to a chemical compound or composition of interest such as a drug treatment, e.g. to test the efficacy and/or toxicity of the drug as previously explained.

Currently cancerous cell lines are cultured in standard well cell plates or culture flasks which are optimized for cell adhesion and proliferation. The surface of the vessels is treated such that cell attachment and proliferation is possible. Numerous coating recipes and methods may be employed. Coatings can consist of extracellular matrix proteins such as collagen, gelatin, fibronectin, laminin and vitronectin, or mixtures thereof. In addition to proteins, chemical and synthetic coatings could be applied such as poly-lysin or poly-omithine. For example, Nunc^{™} plates, which are typically used for cell culturing, are treated with an electrical plasma. This plasma introduces charged, hydrophilic groups on the plastic surface, such as hydroxyl (-OH) or carboxyl (-COOH) groups which improve cell adhesion. The bottoms of these wells are flat, so the cells can only grow in two dimensions, instead of the three-dimensional growth environment that is provided in vivo.

For organ-on-chip applications, three-dimensional, in contrast to two-dimensional growth on a flat surface, is necessary to grow, for example, double cell layers, which mimic organs such as; gut, kidney, liver, lung, skin or the blood-brain-barrier. These last examples can be very well accommodated using membranes arranged between two fluidic flows: an upper fluidic flow to which an upper side of the membrane is exposed, and a lower fluidic flow to which a lower side of the membrane is exposed.

For other organ-on-chip applications micro wells may be needed, with or without a membrane on the bottom of the micro well. Micro wells have dimensions of several 100 µm, for example a diameter of 800 µm and a depth of 500 µm. In these micro wells, spheroids or organoids can be formed and studied. Owing to the fact that the location of a single spheroid or organoid is known, the growth can be followed over time for each individual spheroid or organoid.

Fluidic devices with membranes are known, for example the fluidic devices offered by Emulate Bio. Such devices may, however, have several disadvantages, since the handling protocols may be rather cumbersome.

### SUMMARY OF THE INVENTION

The invention is defined by the claims.

According to examples in accordance with a first aspect of the present disclosure, there is provided a system for use in testing biological material, the system comprising: a substrate; and at least one support member comprising a biocompatible support portion for supporting the biological material, the at least one support member being mountable to, and detachable from, the substrate, wherein the at least one support member is detachable from the substrate at least by manual manipulation by a user.

Such manual manipulation by the user may, for example, involve the user pulling the at least one support member from the substrate using a hand tool, e.g. tweezers or forceps.

Manual detachment of the support member(s) may have various practical benefits, such as facilitating use of a substrate that does not need to have an elaborate fluidic arrangement for supplying fluid to, for example, both sides of the biocompatible support portion, (ii) assisting assessment the biological material without the constraints imposed by the support member(s) being still mounted to the substrate, and/or (iii) facilitating reconfiguration of the system to adjust conditions, e.g. cell culture conditions, to which the biological material is exposed.

It is noted that the support member(s) may be detachable in ways other than the manual manipulation, such as by a robot pulling the support member(s) from the substrate. However, the term "at least by manual manipulation by a user" is intended to mean that the user is able to perform manual manipulation of the system to effect detachment of the support member(s) from the substrate, in addition to such other ways of detaching the support member(s) from the substrate.

In some embodiments, the at least one support member comprises a manipulation portion that is manipulable, for example manipulable via the hand tool, e.g. tweezers or forceps, to permit the detachment of the support member(s) from the substrate.

The manipulation portion can have any suitable design. In some embodiments, the manipulation portion comprises, e.g. is, a rim member or ledge member arranged around at least part of a periphery of the biocompatible support portion.

In more general terms, the manipulation portion, e.g. rim member or ledge member, may be arranged adjacent to the biocompatible support portion.

This arrangement can assist the detachment of the support member(s) with no, or only minimal, interference with the biological material supported on the biocompatible support portion, since it is the manipulation portion, rather than the biocompatible support portion, that is manipulated during the detachment of the support member(s) from the substrate.

In some embodiments, the manipulation portion, e.g. the rim member or ledge member, may be offset with respect to a periphery of the at least one support member, so as to provide a space between the periphery and the manipulation portion.

In such embodiments, at least part of a hand tool, e.g. tweezers or forceps, may be inserted into the space to effect the manual manipulation used to detach the support member(s) from the substrate.

More generally, the support member(s) may be configured so that the biological material remains supported by the biocompatible support portion during the detachment of the at least one support member from the substrate. For instance, manual detachment of the support member(s) may enable relatively straightforward transfer of the biological material that is supported on the biocompatible support portion from one cell culture environment to another cell culture environment.

In some embodiments, the system comprises an orientation indicator system configured to guide mounting of each of the at least one support member in a defined reference orientation relative to the substrate. The orientation indication system can assist to ensure reproducible orientation of the support member with respect to the substrate. This may be beneficial for retrieving and reassessing/remeasuring locations of the biological material, e.g. with an optical system, such as a microscope, after detachment and re-mounting of the support member to the substrate.

The orientation indication system can have any suitable design provided that it is able to guide mounting in the defined reference orientation. In some embodiments, the orientation indicator system comprises a shape feature, such as a protrusion or recess, of the each of the at least one support member and a further shape feature, such as a recess or protrusion, of the substrate. In such embodiments, the shape feature and the further shape feature fit to each other upon adoption of the defined reference orientation.

The biocompatible support portion may comprise a first surface and a second surface, and the biological material may be supportable on at least one, e.g. both, of the first surface and the second surface.

The first and second surfaces may, in use, correspond to upper and lower surfaces of the biocompatible support portion.

In some embodiments, the system may be switchable between a first configuration and a second configuration. In the first configuration, the at least one support member is mounted to the substrate so that one of the first and second surfaces is facing in a given direction, such as upwards. In such embodiments, the at least one support member is detachable from the substrate (e.g. via the manual manipulation) and re-mountable to the substrate so that the other of the first and second surfaces is facing in the given direction, e.g. upwards, in the second configuration.

It is noted that switching between the first and second configurations may involve inverting the detached support member(s), prior to re-mounting the thus inverted support member(s) to the substrate.

The capability to detach and re-mount the support member(s) to the substrate with the support member(s) being inverted can, for example, enable the biological material to remain supported on one of the first and second surfaces of the biocompatible support portion, and further biological material, e.g. a different cell type compared to that of the biological material, to be supported on the other of the first and second surfaces of the biocompatible support portion.

This way of supporting the biological material and the further biological material can be achieved without, for example, having to invert the substrate (and having to sealingly close the top side of the substrate so that when the substrate is inverted fluid cannot leak therefrom).

The design of the support member(s), and in particular the biocompatible support portion thereof, may depend on the nature of the biological material and/or the testing to be carried out. In some embodiments, the biocompatible support portion comprises a biocompatible membrane on which the biological material is supportable. The biocompatible membrane may, for example, have a thickness less than 50 µm, preferably less than 20 µm.

In some embodiments, the biocompatible support portion, e.g. the biocompatible membrane, delimits apertures for allowing fluid to pass therethrough to reach the biological material supported by the biocompatible support portion.

The apertures may be dimensioned so that single cells cannot pass therethrough. This can help to avoid loss of the biological material from the biocompatible support portion, and can, in certain embodiments, help to avoid cells lost from the biocompatible support portion causing contamination of other biological material being tested using the system.

To this end, the apertures may each have a diameter, or if not circular a largest dimension across the respective aperture, of less than about 10 µm. It is noted that the qualifier "about" in this context may refer to a tolerance of ±2 µm.

In some embodiments, the biocompatible support portion comprises well-like depression(s) defined in a biological material-contacting surface of the biocompatible support portion. Such a support member can be particularly suitable for spheroid and/or organoid investigations. Such well-like depression(s) may, for example, each have a diameter of 2000 µm or less.

In some embodiments, the biocompatible support portion comprises a pair of flexible protrusions, e.g. flexible pillars or posts, for supporting tissue thereon and therebetween. In such embodiments, the system may be employed to test muscle tissue, e.g. cardiac tissue, grown between and around the pair of protrusions. When the muscle tissue contracts, the pair of protrusions may deflect or bend towards each other, with displacement of the protrusions caused by the deflection being measurable to enable determination of one or more contraction properties such as for example an extent of contraction, a frequency of contraction and/or a force of contraction provided by contraction of the tissue.

The substrate can have any suitable design. In some embodiments, at least one chamber unit is defined in the substrate, and the at least one support member is mountable to the substrate via insertion of at least part of the at least one support member into one of the chamber unit(s). In such embodiments, the at least one support member may be detachable, at least by said manual manipulation, from the substrate via withdrawal of the at least part of the at least one support member from said one of the chamber unit(s).

In some embodiments, the biocompatible support portion separates an upper portion from a lower portion of the respective chamber unit when the at least part of the support member(s) is inserted into said one of the chamber unit(s).

Fluid, such as cell culture fluid, may be received in the chamber unit via the upper and/or lower portions of the chamber unit. For example, a pipette arrangement may be used for fluid delivery into and/or fluid withdrawal from the at least one chamber unit, e.g. via the upper portion of the chamber unit.

Alternatively or additionally, a flow control system can be used to supply the fluid to, and remove fluid from, the at least one chamber unit. For example, the flow control system may supply the fluid to the upper portion of the chamber unit, and withdraw fluid from the lower portion of the chamber unit.

The chamber unit itself can have any suitable design. In some embodiments, the chamber unit is defined by a single chamber. In other embodiments, multiple chambers, that are fluidly connected with each other via bridge(s) connecting the chambers to each other, can be included in one of the chamber unit(s). In such embodiments, the at least part of the support member can be received in one or more of the multiple chambers.

In some embodiments, a dual chamber unit is included in one or more of the chamber unit(s), with said dual chamber unit comprising a first chamber and a second chamber fluidly connected to each other via a bridge defined between the first chamber and the second chamber. In such embodiments, the at least part of the at least one support member may be insertable into the first chamber and/or the second chamber.

Such a dual chamber unit may have utility in studying metastasis of cancerous cells. It may be possible to assess cells that are released or emitted by any cell cultures that are provided in the first chamber. The first chamber can therefore be used to perform a treatment on the cell culture(s), such as the application of a certain therapeutic fluid or drug, for example a chemotherapy treatment fluid, with the second chamber capturing any released cells to analyze the effect of the treatment performed in the first chamber on metastasis.

The at least one chamber unit may comprise a plurality of chamber units defined in the substrate. In embodiments in which the chamber unit is defined by a single chamber, the plurality of chamber units may correspond to a plurality of individual chambers. In embodiments in which the chamber unit is a dual chamber unit, the plurality of chamber units may correspond to a plurality of dual chamber units.

In some embodiments, the at least one support member comprises a plurality of support members. For example, two or more support members of the plurality of support members may be mountable to the substrate by receiving at least part of each of the two or more support members in said one of the at least one chamber unit. Thus, the two or more support members can, for example, be stacked on top of each other in one chamber unit.

Biopsies, tissue or cell layers can, for example, be retained between the stacked support members. Such a stacked arrangement of the support members can mean that at least one of the two or more support members can, for instance, act as a cover or lid for closing the chamber unit.

The biocompatible support portion may comprise an elastomeric material that provides flexibility to the biocompatible support portion. The flexibility of such a biocompatible support portion can mean that the biocompatible support portion mimics a mechanical environment of the biological material in vivo. This can help to encourage, for instance, cell proliferation.

In some embodiments, the elastomeric material comprises at least one selected from the group consisting of silicone and polybutadiene. These elastomeric materials have been found to function particularly effectively in terms of conferring flexibility to the biocompatible support portion, and have also been found to be relatively straightforward to form into different shapes of the biocompatible support portion, which shapes depend, for example, on the nature of the biological material and/or the type of testing that is to be carried out.

The elastomeric material may, for example, be a fatty acid-crosslinked elastomeric material, for example a fatty acid-crosslinked silicone and/or a fatty acid-crosslinked polybutadiene. Such a fatty acid-crosslinked elastomeric material can be particularly suitable for the biocompatible support portion, owing to the polarity introduced by the carboxylic acid groups of the fatty acid.

Particular mention is made of the support member(s) comprising silicone.

Silicone may be optically transparent and also have limited autofluorescence so that such silicone-based support member(s) may allow a variety of conventionally used optical inspection techniques and protocols, e.g. with or without staining, to be used in testing performed using the system.

In some embodiments the biocompatible support portion comprises a cell culture protein for contacting the biological material. The cell culture protein, e.g. fibronectin, may be arranged, for instance, on surface(s) of the fatty acid-crosslinked elastomeric material at which the carboxylic acid groups are arranged.

According to examples in accordance with a second aspect of the present disclosure, there is provided a use of the system according to any of the embodiments described herein for testing biological material.

In some embodiments, the biological material being tested comprises one or more chosen from the group consisting of: cell groups, double cell layers, spheroids, organoids and biopsies.

According to examples in accordance with a third aspect of the present disclosure, there is provided a method of testing biological material, the method comprising: supporting the biological material on the biocompatible support portion included in the at least one support member of the system according to any of the embodiments described herein, the at least one support member being mounted to the substrate; optionally wherein said supporting biological material comprises culturing cells on the biocompatible support portion; and manually manipulating the at least one support member to detach the at least one support member from the substrate.

In some embodiments, the method comprises exposing the biological material to a drug to be tested.

In such embodiments, the method may further comprise monitoring a response of the biological material, e.g. cultured cells, to the drug to be tested. This monitoring may be implemented in any suitable manner, for example by optical microscopy.

According to examples in accordance with a fourth aspect of the present disclosure, there is provided a support member for supporting biological material, the support member being mountable to, and detachable from, a substrate, the support member comprising a biocompatible support portion for supporting the biological material.

The support member may be according to any of the embodiments described herein in relation to the first, second and third aspects.

These and other aspects of the invention will be apparent from and elucidated with reference to the embodiments described hereinafter.

### BRIEF DESCRIPTION OF THE DRAWINGS

For a better understanding of the invention, and to show more clearly how it may be carried into effect, reference will now be made, by way of example only, to the accompanying drawings, in which:
FIGs. 1A to 1G schematically depict different configurations of a system according to a first example;
FIGs. 2A and 2C schematically depict different configurations of a system according to a second example, with FIG. 2B showing a plan view of a substrate included in the system;
FIG. 2D schematically depicts a support member according to an example;
FIG. 2E schematically depicts a support member according to another example;
FIGs. 3A and 3B provide perspective views of a support member of a system according to a third example;
FIG. 4 provides views and illustrative dimensions of a support member of a system according to a fourth example;
FIGs. 5A to 5C schematically depict different configurations of a system according to a fifth example;
FIG. 6 provides a perspective view of a system according to a sixth example;
FIG. 7 provides a perspective view of a system according to a seventh example;
FIG. 8 provides a more detailed view of the substrate included in the system shown in FIG. 7; and
FIG. 9 provides a perspective view of a substrate of a system according to an eighth example.

### DETAILED DESCRIPTION OF THE EMBODIMENTS

The invention will be described with reference to the Figures.

The detailed description and specific examples, while indicating exemplary embodiments of the apparatus, systems, substrates and support members as well as the methods of their use and methods of their manufacture, are intended for purposes of illustration only and are not intended to limit the scope of the invention. These and other features, aspects, and advantages of the apparatus, systems and methods of the present invention will become better understood from the following description, appended claims, and accompanying drawings. The Figures are merely schematic and are not drawn to scale. The same reference numerals are used throughout the Figures to indicate the same or similar parts.

Provided is a system for use in testing biological material. Testing of biological material in this context can mean for example the growing, cultivating, and differentiating of cells and cultures of cells such as for example spheroids and organoids. The system includes a substrate, and at least one support member that has a biocompatible support portion for supporting the biological material. The at least one support member is mountable to, and detachable from, the substrate. The at least one support member is detachable from the substrate at least by manual manipulation by a user, for example by the user pulling the at least one support member from the substrate by manual operation of a hand tool, e.g. tweezers or forceps.

Additionally provided is the support member per se.

Further provided is a method of testing biological material using the system, as well as use of such a system for testing biological material.

Examples of how cell differentiation can be implemented can be found in the patent application published as WO2020148427 which is incorporated by reference in its entirety herein.

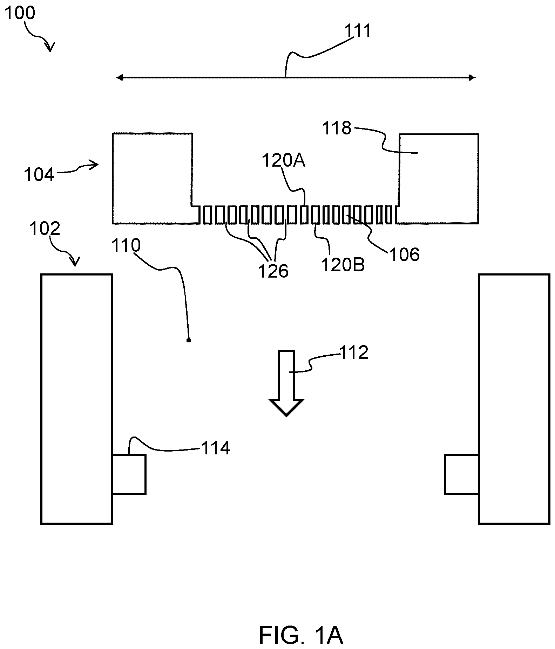

FIG. 1A schematically depicts a system 100 for testing biological material according to an example. The system comprises a substrate 102 and at least one support member 104. The, e.g. each of the, at least one support member 104 comprises a biocompatible support portion 106 on which the biological material is supportable.

The biological material may include, for example, at least one selected from the group consisting of cell groups, double cell layers, spheroids, organoids and biopsies.

Biological material tends to be required to be living during at least part of the testing performed using the system 100. Hence the term "biocompatible" in the context of the biocompatible support portion 106 may mean that the biological material is permitted to live during contact with the biocompatible support portion 106.

The biocompatible support portion 106 may comprise an elastomeric material that provides flexibility to the biocompatible support portion 106. The flexibility of such a biocompatible support portion 106 can mean that the biocompatible support portion 106 mimics a mechanical environment of the biological material in vivo. This can help to encourage, for instance, cell proliferation.

The material, e.g. elastomeric material, of the biocompatible support portion 106 may be suitable for at least one of: cell culture, spheroid formation, organoid formation and propagation, and maintaining tissue integrity.

In some embodiments, the elastomeric material comprises at least one selected from the group consisting of silicone and polybutadiene. These elastomeric materials have been found to function particularly effectively in terms of conferring flexibility to the biocompatible support portion 106, and have also been found to be relatively straightforward to form into different shapes of the biocompatible support portion 106, which shapes depend, for example, on the nature of the biological material and/or the type of testing that is to be carried out.

Particular mention is made of the biocompatible support portion 106 comprising silicone.

Silicone may be optically transparent and also have limited autofluorescence so that the silicone-based biocompatible support portion 106 may allow a variety of conventionally used optical inspection techniques and protocols, e.g. with or without staining, to be used in testing performed using the system 100.

The elastomeric material may, for example, be a fatty acid-crosslinked elastomeric material, for example a fatty acid-crosslinked silicone and/or a fatty acid-crosslinked polybutadiene. Such a fatty acid-crosslinked elastomeric material can be particularly suitable for the biocompatible support portion 106, owing to the polarity introduced by the carboxylic acid groups of the fatty acid.

The fatty acid-crosslinked elastomeric material can be manufactured in any suitable manner, such as by bulk-modifying the elastomeric material with the fatty acid. It is noted that bulk-modified elastomeric materials suitable for inclusion in the biocompatible support portion 106 are described, for example, in WO2021058657 and WO2019015988.

In some embodiments, at least some of the carboxylic acid groups of the fatty acids crosslinked to the elastomeric material, e.g. to the silicone and/or polybutadiene, are arranged at surface(s) of the fatty acid-crosslinked elastomeric material.

In such embodiments, the fatty acids included in the fatty acid-crosslinked elastomeric material may render the surface(s) of the elastomeric material particularly compatible with polar species, such as cell culture protein(s), e.g. fibronectin, and/or a polar environment to which the biological material is exposed during the testing.

In some embodiments the biocompatible support portion 106 comprises a cell culture protein for contacting the biological material. The cell culture protein, e.g. fibronectin, may be arranged, for instance, on surface(s) of the fatty acid-crosslinked elastomeric material at which the carboxylic acid groups are provided.

It is noted that in some embodiments the, e.g. each of the, at least one support member 104 is entirely formed of a biocompatible material, such as a biocompatible material comprising the fatty acid-crosslinked silicone and/or the fatty acid-crosslinked polybutadiene.

In other embodiments, the biocompatible support portion 106 is formed of a biocompatible material while other portion(s) of the respective support member 104 is/are formed from other material(s), such as non-biocompatible material(s).

In such embodiments, the biological material may reside on the biocompatible support portion 106 rather than on the portion(s) that are formed from the other, e.g. non-biocompatible, material(s).

The support member(s) 104 can be fabricated using any suitable manufacturing technique, such as via molding, e.g. injection molding, or casting. For example, the support member(s) 104 can be fabricated by injection molding a slab, such as a silicone or polybutadiene slab, comprising a plurality of support members 104, and then isolating each of the support members 104. The latter can be achieved, for instance, by punching out each of the support members 104 from the slab. Alternatively, the support member(s) 104 can be molded directly, without having to be punched from a slab.

The substrate 102 can be formed from any suitable material, for example from a metallic material, e.g. stainless steel, a glass, a machinable glass ceramic, and/or a plastic material, such as a thermosetting plastic material. In some embodiments, the substrate 102 comprises at least one selected from the group consisting of polycarbonate and polystyrene.

The substrate 102 can, for example, comprise a thermoplastic, an elastomer, e.g. a thermoplastic elastomer.

It has, for example, been proven that a printed substrate 102 formed from a thermoplastic elastomer, e.g. Cawiton, can be reused after oncology testing, following disinfecting with isopropyl alcohol.

The substrate 102 can be manufactured using any suitable technique, such as via additive manufacturing, e.g. 3D printing, or molding, such as injection molding.

In some embodiments, the substrate 102 is formed from a rigid plastic material and the support member 104, e.g. at least the biocompatible support portion 106 thereof, comprises the elastomeric material.

In such embodiments, the substrate 102 may, for instance, be formed of the rigid plastic material, and the support member 104, e.g. at least the biocompatible support portion 106 thereof, comprises silicone as the elastomeric material.

In a non-limiting example, the substrate 102 is formed of the rigid plastic material in the form of a thermoplastic material, and the support member 104, e.g. at least the biocompatible support portion 106 thereof, comprises silicone as the elastomeric material.

In another non-limiting example, the substrate 102 is formed of the rigid plastic material in the form of a printed material, and the support member 104, e.g. at least the biocompatible support portion 106 thereof, comprises silicone as the elastomeric material.

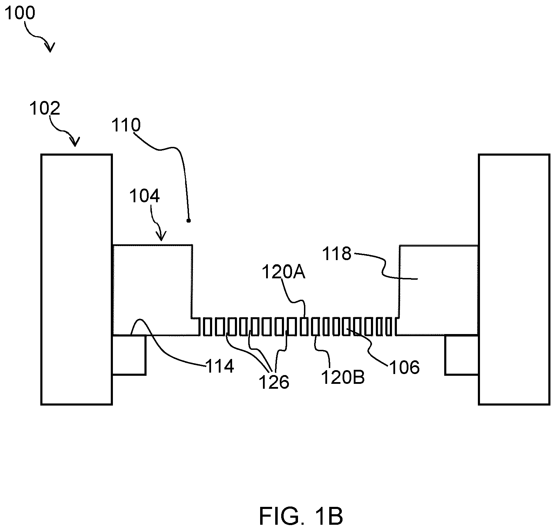

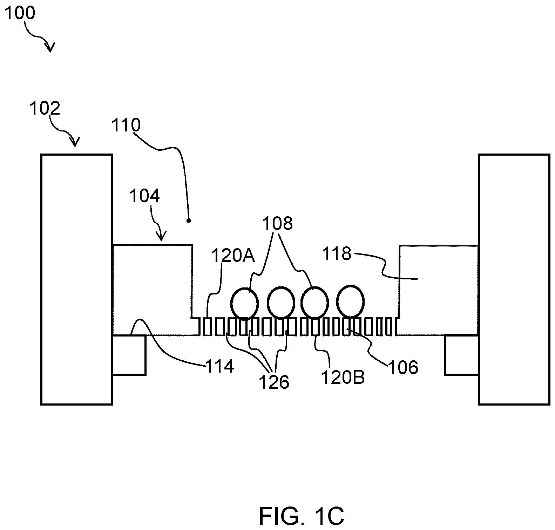

More generally, and referring to FIGs. 1A and 1B, the at least one support member 104 is mountable to the substrate 102. As shown in FIG. 1C, the biological material 108 may be supported on the biocompatible support portion 106 while the at least one support member 104 is mounted to the substrate 102.

The support member(s) 104 can be mounted to the substrate 102 in any suitable manner. In some embodiments, such as shown in FIGs. 1A to 1C, at least one chamber unit 110 is defined in the substrate 102, and the at least one support member 104 is mountable to the substrate 102 via insertion of at least part of the at least one support member 104 into one of the chamber unit(s) 110.

The at least part of the support member(s) 104 that is insertable into the chamber unit 110 may include the biocompatible support portion 106.

Thus, the biological material 108 may be supported on the biocompatible support portion 106 and within the chamber unit 110 while the support member(s) 104 is/are mounted to the substrate 102.

The chamber unit(s) 110 may be configured to receive fluid, such as cell culturing fluid, therein.

Accordingly, the biological material 108 may receive such fluid, e.g. cell culturing fluid, while the biological material 108 is supported by the biocompatible support portion 106 within the chamber unit 110.

Insertion of the at least part of the support member(s) 104 into the chamber unit 110 may comprise, for example, lowering the at least part of the support member(s) 104 into the chamber unit 110 (see the arrow 112 in Fig. 1A) so that the at least part of the support member(s) 104 is received into the chamber unit 110 from above. Alternatively, the at least part of the support member(s) 104 may be raised into the chamber unit 110 from beneath the substrate 102, or the substrate 102 may be lowered onto the support member(s) 104 so that the at least part of the support member(s) 104 is inserted into the chamber unit(s) 110 (not shown).

An outer dimension of the at least one support member 104 may be tuned so that the at least one support member 104 precisely fits into the chamber unit 110 into which the at least one support member 104 is to be inserted.

In some embodiments, such as shown in FIGs. 1A to 1C, the substrate 102 comprises at least one supporting region 114 for supporting the at least one support member 104 when the at least part of the support member(s) 104 is inserted into the chamber unit 110. The supporting region(s) 114 can take any suitable form, such as a rim or ledge located within the chamber unit(s) 110, on which rim or ledge the support member(s) 104 can rest.

In other embodiments, the supporting region(s) 114 can be omitted, for instance with the support member 104 being placeable at the bottom of the chamber unit 110.

In some embodiments, and referring to FIGs. 1B and 1C, the biocompatible support portion 106 separates an upper portion from a lower portion of the respective chamber unit 110 when the at least part of the support member(s) 104 is inserted into said one of chamber unit(s) 110. For example, the biocompatible support portion 106 separates the upper portion from the lower portion when the at least one support member 104 is supported by the supporting region(s) 114.

Fluid, such as cell culture fluid, may be received in the chamber unit 110 via one of the upper and lower portions of the chamber unit 110 and exit the chamber unit 110 via the other of the upper and lower portions of the chamber unit 110.

For example, a pipette arrangement may be used for fluid delivery into and/or fluid withdrawal from the at least one chamber unit 110, e.g. via the upper portion of the chamber unit 110.

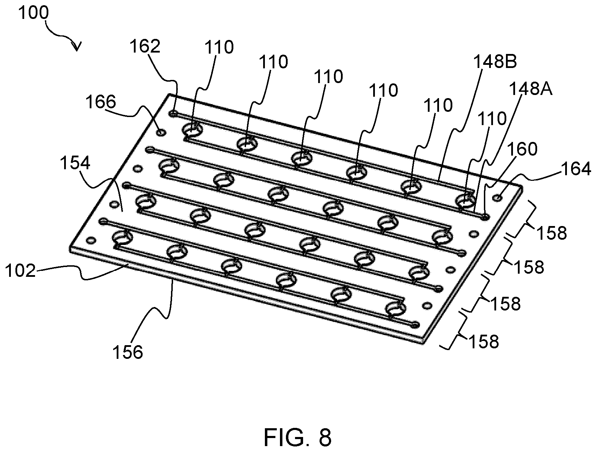

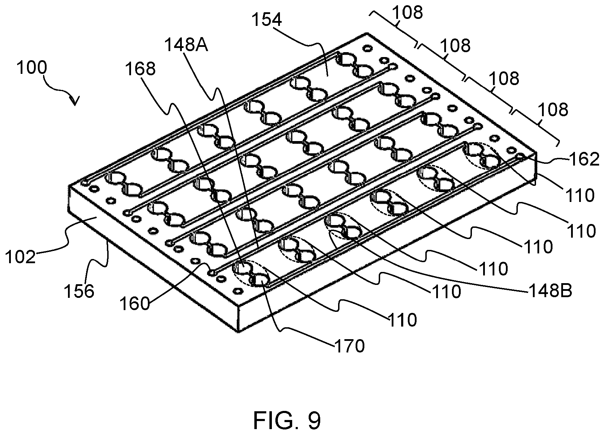

Alternatively or additionally, a flow control system (not visible) can be used to supply the fluid to, and remove fluid from, the at least one chamber unit 110. Non-limiting examples of substrates 102 that can be used in conjunction with such a flow control system are described herein below with reference to Figs. 8 and 9.

It is noted that flow control systems for supplying fluid to, and removing fluid from, chamber unit(s) 110 defined in the substrate 102 are commercially available, for example from Fluigent^{®}.

The chamber unit(s) 110 may have dimensions suitable for the intended testing of the biological material 108. In some embodiments, each chamber unit 110 has a height extending between a top side of the substrate 102 and a bottom side of the substrate 102 of 1.8 to 2.6 cm, e.g. about 2.2 cm. Alternatively or additionally, a diameter of each chamber unit 110 may be 3 to 10 mm, e.g. about 6 mm.

Such dimensions may balance the requirement for the chamber unit(s) 110 to be kept as small as possible, e.g. to enable as many chamber unit(s) 110 to be defined in the substrate 102 as possible, with the requirement for the chamber unit(s) 110 to provide sufficient space for testing of the biological material 108, e.g. for capture and subsequent analysis of cells.

The diameter 111 of the support member 104, or at least the part of the support member 104 that is received in the chamber unit 110, may be selected to enable fitting inside the chamber unit 110. For example, the diameter 111 of the support member 104 shown in FIGs. 1A to 1C is about 6 mm, which corresponds to an approximately 6 mm diameter chamber unit 110.

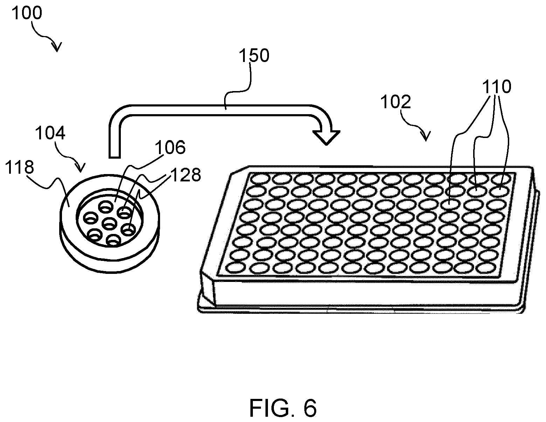

The substrate 102 may, for instance, have a standardized design, such as a 96 well/chamber unit 110 cell plate (e.g. with the wells having a diameter of about 6 mm). Such an example of a standardized substrate 102 is described herein below with reference to FIG. 6.

In some embodiments, two or more support members 104 are mountable to the substrate 102 by receiving at least part of each of the two or more support members 104 in said one of the chamber unit(s) 110. Thus, the two or more support members 104 can, for example, be stacked on top of each other in the one chamber unit 110. Biopsies, tissue or cell layers can, for example, be retained between the stacked support members 104. Such a stacked arrangement of the support members 104 can mean that at least one of the two or more support members 104 can, for instance, act as a cover or lid for closing the chamber unit 110.

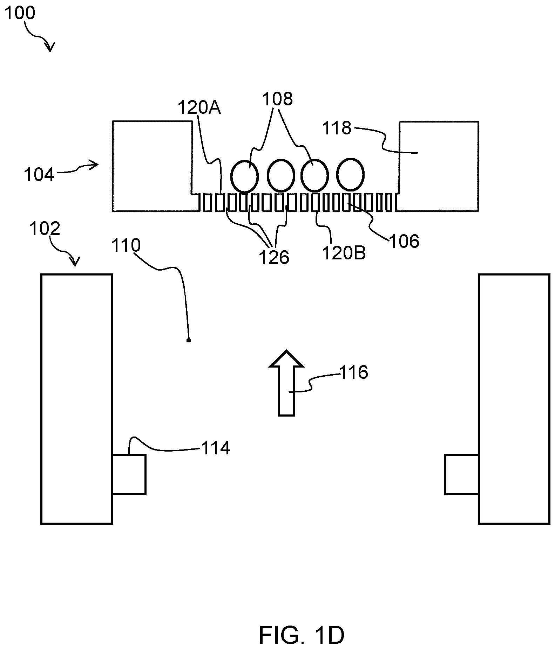

More generally, and referring now to FIG. 1D, the at least one support member 104 is detachable from substrate 102, at least by manual manipulation by a user. Such manual manipulation by the user may, for example, involve the user pulling the at least one support member 104 from the substrate 102 using a hand tool, e.g. tweezers or forceps. The hand tool may be sterile.

Manual detachment of the support member(s) 104 may have various practical benefits, such as (i) facilitating use of a substrate 102 that does not need to have an elaborate fluidic arrangement for supplying fluid to, for example, both sides of the biocompatible support portion 106, (ii) assisting assessment of the biological material 108 without the constraints imposed by the support member(s) 104 being still mounted to the substrate 102, and/or (iii) facilitating reconfiguration of the system 100 to adjust conditions, e.g. cell culture conditions, to which the biological material 108 is exposed. A non-limiting example of the latter is explained in more detail herein below, with reference to Figs. 1A to 1G.

Regarding (ii), manual detachment of the support member(s) 104 can, for example, facilitate various analyses, including, but not limited to, RNA-, DNA- or whole genome sequencing.

Turning to (iii), manipulating biocompatible support portions 106 in the form of membranes while culturing cells, e.g. to form organoids or cell (barrier) layers, can be problematic. The initial cells cultured in a certain medium tend to be added to the membrane from above so that cells can sediment onto an upper side of the membrane by gravitational forces. Because most, if not all, organ-on-chip systems are closed fluidic systems, the underside of the membrane may be difficult to reach for cells to attach onto the membrane. It follows that the capability to manually detach the support member(s) 104 from the substrate 102 can facilitate access to the underside of the membrane.

For example, detachment of the support member(s) 104 can enable the underside of the membrane to be seeded with a different cell type, as explained in more detail herein below.

It is noted that the support member(s) 104 may be detachable in ways other than said manual manipulation, such as by a robot pulling the support member(s) 104 from the substrate 102. However, the term "at least by manual manipulation by a user" is intended to mean that the user is able to perform manual manipulation of the system 100 to effect detachment of the support member(s) 104 from the substrate 102, in addition to such other ways of detaching the support member(s) 104 from the substrate 102.

Implicit in the detachability of the at least one support member 104 from the substrate 102 is that the at least one support member 104 is sufficiently weakly interacting with the substrate 102 that the user is able to manually detach the at least one support member 104 from the substrate 102.

Such manual detachment may be precluded if, for example the at least one support member 104 were to be glued to the substrate 102, since adhesion provided by the glue may be sufficiently strong to prevent the user from detaching the at least one support member 104 from the substrate 102 using his/her hand(s), even when his/her hand(s) is/are operating a hand tool, e.g. tweezers or forceps.

Such manual detachment may also be precluded if, for example, the at least one support member 104 were to be wedged in a portion of the substrate 102, such as wedged in the chamber unit 110, so tightly that the user cannot detach the at least one support member 104 from the substrate 102 using his/her hand(s), even when his/her hand(s) is/are operating a hand tool, e.g. tweezers or forceps.

In embodiments in which the at least one support member 104 is mountable to the substrate 102 via insertion of at least part of the support member(s) 104 into one of the at least one chamber unit 110, the detachment of the support member(s) 104 from the substrate 102 may comprise withdrawing the at least part of the support member(s) 104 from said one of the at least one chamber unit 110, for example by the user using a hand tool, e.g. tweezers or forceps, to pull the support member(s) 104 out of the respective chamber unit 110 (see the arrow 116 in FIG. 1D).

In some embodiments, the at least one support member 104 comprises a manipulation portion 118 that is manipulable, for example manipulable via the hand tool, e.g. tweezers or forceps, to permit the detachment of the support member 104 from the substrate 102.

The manipulation portion 118 can have any suitable design. In some embodiments, such as shown in FIGs. 1A to 1G, the manipulation portion 118 comprises, e.g. is, a rim member or ledge member arranged around at least part of a periphery of the biocompatible support portion 106.

It is noted that the manipulation portion 118, e.g. rim member or ledge member, of the at least one support member 104 can, for example, be formed from the same material as used to form the biocompatible support portion 106, such as the elastomeric material, e.g. silicone and/or polybutadiene.

The manipulation portion 118, e.g. rim member or ledge member, may be flexible but have sufficient rigidity to be manipulated. For example, cells cultured on the biocompatible support portion 106 may remain attached thereto during the manipulation of the manipulation portion 118, for example during detachment from the substrate 102 and transfer into a fluidic device.

In some embodiments, an adhesion force between a periphery of the at least one support member 104, e.g. a periphery of the rim member, and a wall of the substrate 102 is sufficient to prevent leakage between the at least one support member 104 and the wall of the substrate 102. However, the adhesion force is not so strong as to preclude the detachment of the support member(s) 104 from the substrate 102 via (at least) the manual manipulation.

The manipulation portion 118, e.g. rim member or ledge member, may be arranged adjacent to the biocompatible support portion 106. This arrangement can assist the detachment of the support member(s) 104 with no, or only minimal, interference with the biological material 108 supported on the biocompatible support portion 106, since it is the manipulation portion 118, rather than the biocompatible support portion 106, that is manipulated during the detachment of the support member(s) 104 from the substrate 102.

This can be advantageous when, for example, a purpose of detaching the support member(s) 104 from the substrate 102 is to facilitate assessment of the biological material 108 since the biological material 108 is less likely to be interfered with as a consequence of the detachment of the support member(s) 104 from the substrate 102.

In more general terms, and referring again to FIG. 1D, the support member(s) 104 may be configured so that the biological material 108 remains supported by the biocompatible support portion 106 during the detachment of the at least one support member 104 from the substrate 106.

The biocompatible support portion 106 may comprise a first surface 120A and a second surface 120B on which the biological material 108 is supportable. The first and second surfaces 120A, 120B may, in use, correspond to upper and lower surfaces of the biocompatible support portion 106.

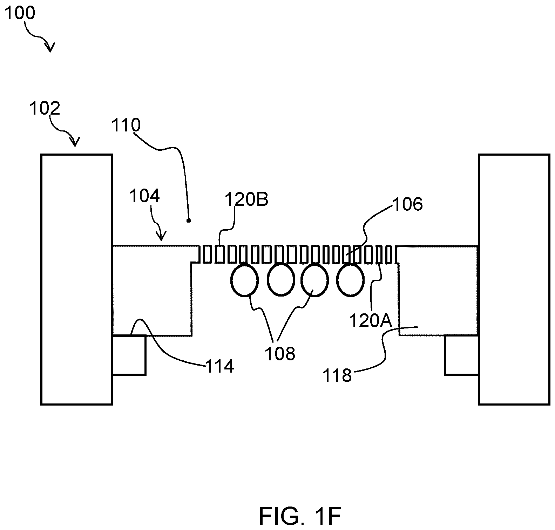

In some embodiments, and referring to FIGs. 1C to 1F, the system 100 may be switchable between a first configuration (see FIG. 1C) and a second configuration (see FIG. 1F). In the first configuration, the at least one support member 104 is mounted to the substrate 102 so that one of the first and second surfaces 120A, 120B is facing in a given direction. In such embodiments, the at least one support member 104 is detachable from the substrate 102 (e.g. via the manual manipulation - see FIG. 1D) and re-mountable to the substrate 102 so that the other of the first and second surfaces 120B, 120A is facing in the given direction in the second configuration (see FIG. 1F).

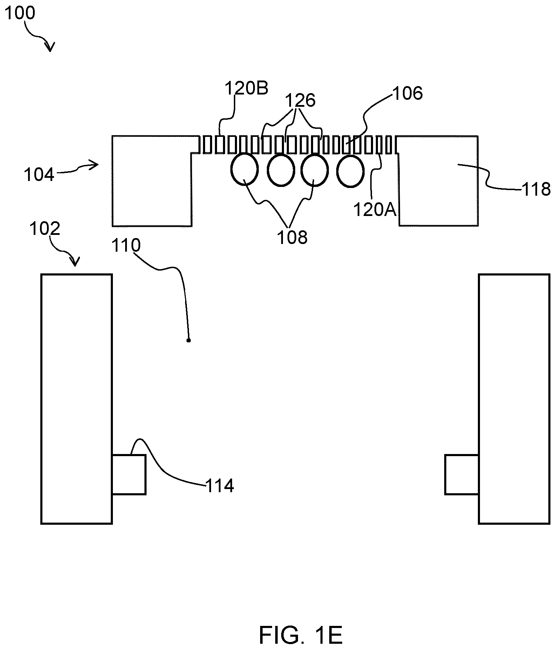

It is noted that switching between the first and second configurations may involve inverting the detached support member(s) 104, as shown in FIG. 1E, prior to re-mounting the thus inverted support member(s) 104 to the substrate 102.

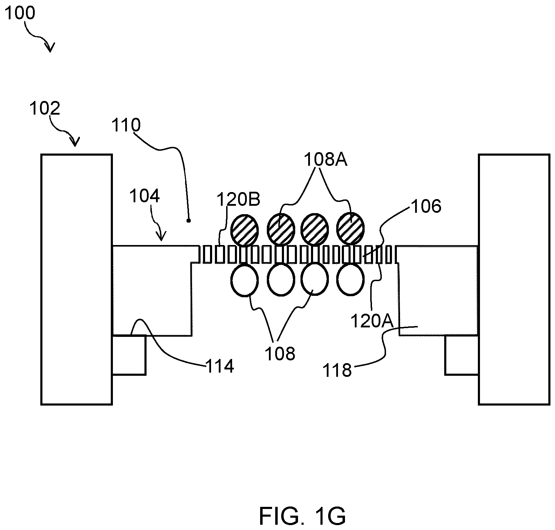

Referring now to FIG. 1G, the capability to detach and re-mount the support member(s) 104 to the substrate 102 with the support member(s) 104 being inverted can, for example, enable the biological material 108 to remain supported on one of the first and second surfaces 120A, 120B of the biocompatible support portion 106, and further biological material 108A, e.g. a different cell type compared to that of the biological material 108, to be supported on the other of the first and second surfaces 120B, 120A of the biocompatible support portion 106.

This way of supporting the biological material 108 and the further biological material 108A can be achieved without, for example, having to invert the substrate 102 (and having to sealingly close the top side of the substrate 102 so that when the substrate 102 is inverted fluid cannot leak therefrom).

In some embodiments, the substrate 102 is a standard well cell plate in one of whose chamber units 110 the biological material 108 in the form of a first layer of cells is cultured on one of the first and second surfaces 120A, 120B of the biocompatible support portion 106 when the system 100 is in the first configuration. When in the second configuration, adopted via inversion of the detached support member 104 and re-mounting of the support member 104 in the chamber unit 110, the further biological material 108A in the form of a second layer of cells, e.g. different cells with respect to the first layer of cells, can be cultured on the other of the first and second surfaces 120B, 120A of the biocompatible support portion 106, so as to provide a double cell layer.

In such embodiments, the support member 104 supporting the biological material 108 and the further biological material 108A in the form of the double cell layer can be detached from the substrate 102, e.g. via the manual manipulation, and, for example, inserted into a fluidic device configured to apply fluids to both of the first and second layers of cells using fluidic flow conditions.

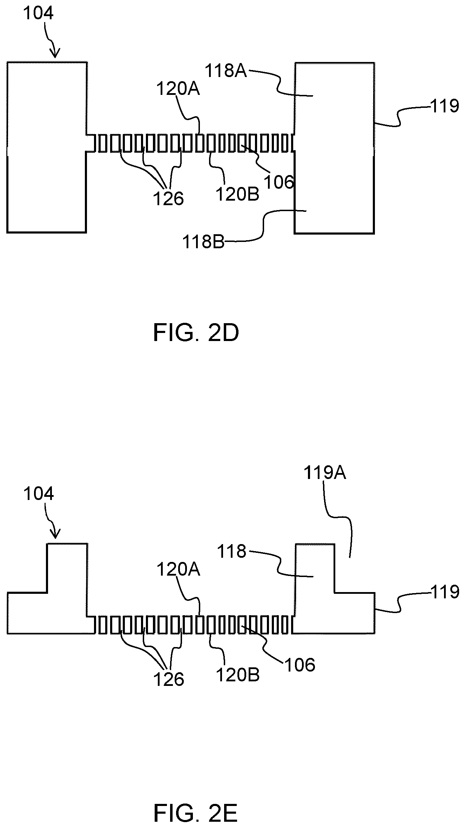

In embodiments in which the system 100 is switchable between the first configuration and the second configuration, and referring to FIG. 2D, the manipulation portion 118 may be arranged to enable the detachment, at least by manual manipulation, of the support member(s) 104 from the substrate 102 in each of the first configuration and the second configuration, for example by a first rim member or first ledge member 118A being provided at one side of the respective support member 104 and a second rim member or second ledge member 118B being provided at a second side of the respective support member 104 that is opposite to the first side.

Alternatively or additionally, and referring now to FIG. 2E, the manipulation portion 118, e.g. the rim member or ledge member, may be offset with respect to a periphery 119 of the at least one support member 104.

Accordingly, a space 119A may be provided between the periphery 119 and the manipulation portion 118, in which space 119A at least part of a hand tool, e.g. tweezers or forceps, may be inserted to effect the manual manipulation used to detach the respective support member from the substrate 102.

In such embodiments, the periphery 119 may still retain engagement, e.g. a seal, against the substrate 102, e.g. against a sidewall of the chamber unit 110 in which the support member 104 is insertable.

In embodiments in which the first rim member or first ledge member 118A is provided at one side of the respective support member 104 and the second rim member or second ledge member 118B is provided at a second side of the respective support member 104 that is opposite to the first side, the first rim member or first ledge member 118A may be offset with respect to the periphery 119 of the support member 104 (so as to provide a space 119A between the first rim member/first ledge member 118A and the periphery 119), and the second rim member or second ledge member 118B may be offset with respect to the periphery 119 of the support member 104 (so as to provide a space between the second rim member/second ledge member 118B and the periphery 119).

In such embodiments, and when the supporting region 114 is included in the substrate 102, the distance between the first rim member/first ledge member 118A and the periphery 119 may be large enough to accommodate the supporting region 114, and/or the distance between the second rim member/second ledge member 118B and the periphery 119 may be large enough to accommodate the supporting region 114.

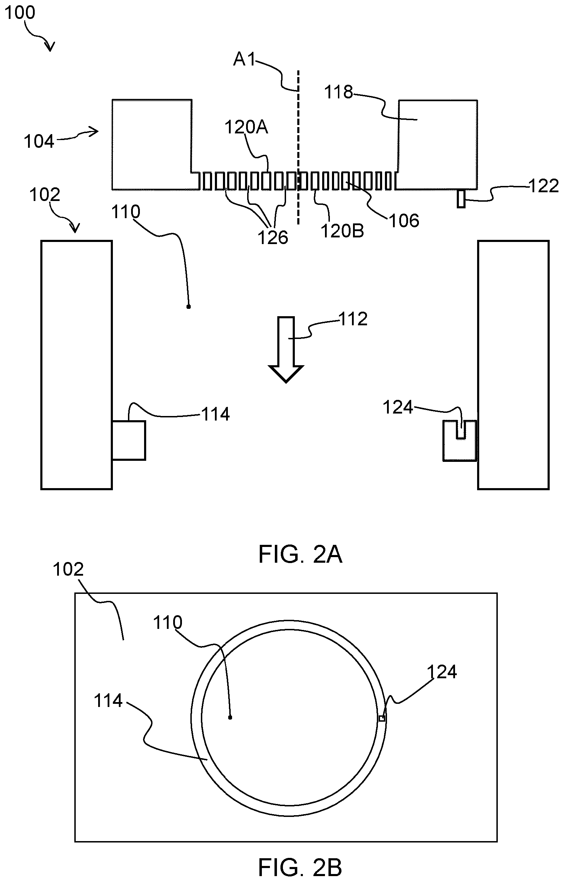

In some embodiments, and referring now to FIGs. 2A to 2C, the system 100 comprises an orientation indicator system 122, 124 configured to guide mounting of each of the at least one support member 104 in a defined reference orientation relative to the substrate 102. The orientation indication system 122, 124 can assist to ensure reproducible orientation of the support member 104 with respect to the substrate 102. This may be beneficial for retrieving and reassessing/remeasuring locations of the biological material 108, e.g. with an optical system, such as a microscope, after detachment and re-mounting of the support member 104 to the substrate 102.

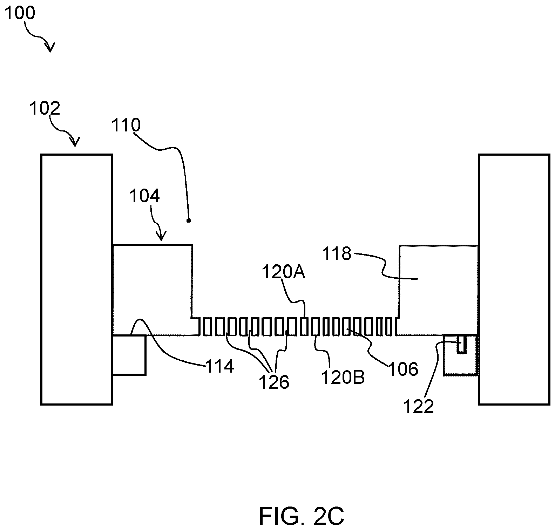

The orientation indication system 122, 124 can have any suitable design provided that it is able to guide mounting in the defined reference orientation. In some embodiments, and still referring to FIGs. 2A to 2C, the orientation indicator system 122, 124 comprises a shape feature 122, such as a projection or recess, of the each of the at least one support member 104 and a further shape feature 124, such as a recess or projection, of the substrate 102. In such embodiments, the shape feature 122 and the further shape feature 124 fit to each other upon adoption of the defined reference orientation, as shown in FIG. 2C.

Referring to FIG. 2B, the further shape feature 124, e.g. recess or projection, may for instance be arranged at a single position around a circumference of the rim defining the supporting region 114, such that the shape feature 122 of the support member 104 can only be fitted to the further shape feature 124 at one angular orientation adoptable by rotating the support member 104 about a central axis A1.

In some embodiments, such as shown in FIGs. 2A and 2B, the shape feature 122, e.g. projection or recess, is arranged at a bottom of the support member 104. In other embodiments, the shape feature 122, e.g. projection or recess, is arranged at a side of the support member 104, with the further shape feature 124, e.g. recess or projection, being arranged at, for example, a sidewall of the substrate 102, e.g. a sidewall of the chamber unit 110 in which the support member 104 is insertable.

In embodiments in which the support member(s) 104 comprise(s) the elastomeric material, the shape feature 122 in the form of the projection at a bottom of the support member 104 may, for instance, be formed from a material that is more rigid than the elastomeric material.

This can assist to mitigate the risk of the projection being insufficiently rigid to be locatable in a recess that defines the further shape member 124 included in the substrate 102.

In some embodiments, the orientation indicator system 122, 124 comprises indicators that can be identified as such, with indicator(s) provided on the support member 104 being alignable with further indicator(s) on the substrate 102.

Such indicator(s) can take any suitable form, such as a pair of arrows, and/or by one or both of the substrate 102 and the support member 104 having a shallow recess in the form of a line defined therein.

It is reiterated at this point that the design of the support member(s) 104, and in particular the biocompatible support portion 106 thereof, may depend on the nature of the biological material 108 and/or the testing to be carried out. In some embodiments, the biocompatible support portion 106 comprises a biocompatible membrane on which the biological material 108 is supportable. The biocompatible membrane may, for example, have a thickness less than 50 µm, preferably less than 20 µm.

In some embodiments, such as shown in FIGs. 1A to 1G, 2A and 2B, the biocompatible support portion 106, e.g. the biocompatible membrane, delimits apertures 126 for allowing fluid to pass therethrough to reach the biological material 108 supported by the biocompatible support portion 106.

The apertures 126 may be dimensioned so that single cells cannot pass therethrough. This can help to avoid loss of the biological material 108 from the biocompatible support portion 106, and can, in certain embodiments, help to avoid cells lost from the biocompatible support portion 106 causing contamination, for instance contamination of other chamber units 110 defined in the substrate 102 when the chamber units 110 are fluidically coupled to each other.

To this end, the apertures 126 may each have a diameter, or if not circular a largest dimension across the respective aperture 126, of less than about 10 µm. It is noted that the qualifier "about" in this context may refer to a tolerance of ±2 µm.

Such apertures 126 can be formed in any suitable manner, for example by laser ablation or casting imprint perforation.

A diameter of the laser pattern employed in the case of laser ablation being used to form the apertures 126 may be, 1 mm to 10 mm, such as 1 mm to 5 mm, e.g. about 3.5 mm. Laser perforation/ablation is described, for example, in US2014127744 and US20180315409. Casting imprint perforation is described, for instance, in US2020360923, US2015010919 and US2022228108.

Whilst not visible in FIGs. 1A to 1G, 2A and 2B, the apertures 126 can have a tapering or V-shaped profile. Alternatively or additionally, the number of apertures 126 may be at least 500, such as at least 1000, e.g. 500 to 5000, such as about 3500.

In a working, non-limiting example, the apertures 126 are formed using laser ablation, using a laser pattern with a diameter of about 3.5 mm, creating 3500 apertures 126 having a tapering/V-shaped profile, and with the diameter of the resulting apertures 126 being about 10 µm.

In some embodiments, the material, e.g. elastomeric material, forming the biocompatible support portion 106 may be manufactured, e.g. by injection molding, as a slab of material that is subsequently subjected to an aperture-forming process, e.g. comprising laser ablation. In this manner, several biocompatible support portions 106 may, for instance, be manufactured in parallel. The biocompatible support portions 106 may then be separated from each other, for example by being punched out of the slab.

In a non-limiting illustrative example, slabs of elastomeric material having a thickness of about 1 mm, a length of about 5 cm and a width of about 5 cm may be formed via injection molding, with the slabs each comprising three biocompatible support portions 106 whose thickness is about 20 µm and whose diameter is about 4 mm. By using a 6 mm punch, three support members 104 can be provided, with each of the support members 104 comprising one of the biocompatible support portions 106 and a manipulation portion 118 in the form of a 2 mm rim.

In such an example, laser ablation may be used to ablate apertures 126, e.g. micro-holes, in each of the three biocompatible support portions 106. The number, dimensions and shape of the apertures 126 may, for instance, be as described in the working, non-limiting example provided above.

In other embodiments, the biocompatible support portion 106 can be molded directly, without having to be punched from a slab.

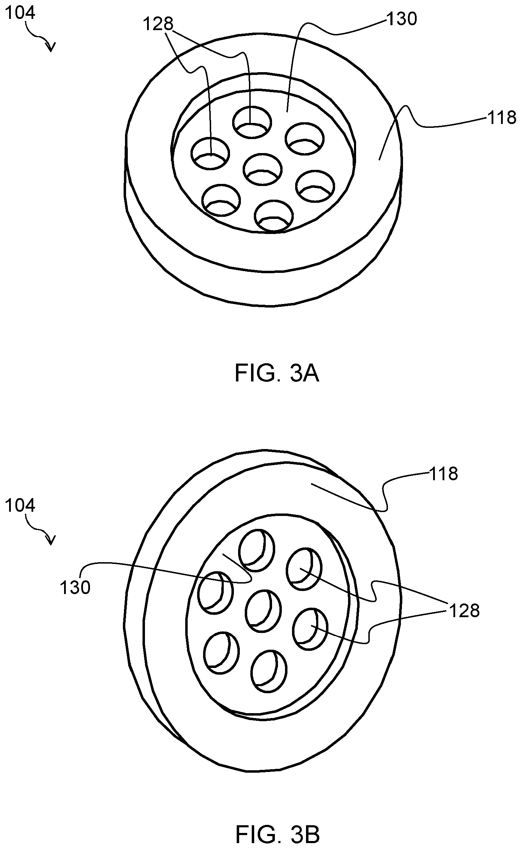

In some embodiments, and referring now to FIGs. 3A and 3B, well-like depression(s) 128 is/are defined in a biological material-contacting surface 130 of the biocompatible support portion 106. Such a support member 104 can be particularly suitable for spheroid and/or organoid investigations. Such well-like depression(s) 128 may, for example, have a diameter of 2000 µm or less and/or a depth at a deepest point of the respective well-like depression 128 of at least 50 µm. The well-like depression(s) 128 can, for instance, be regarded as "micro well(s)".

Seven well-like depressions 128 are provided in the non-limiting example shown in FIGs. 3A and 3B. However, the number of well-like depressions 128 is not particularly limited, and could be one, two, three, four, five, six, eight, nine, ten, or more, such as about twenty, about thirty, about forty, about fifty, about sixty or about seventy.

In a non-limiting illustrative example, fifty seven well-like depressions 128 are provided, with each of the well-like depressions 128 having a diameter of about 150 µm and a depth of 50 to 100 µm.

In some embodiments, a base surface at a base/deepest point of the well-like depression(s) 128 may be a smooth surface. The latter may be achieved via, for instance, a high-gloss polished surface of the mold used for injection molding the biocompatible support portion 106.

Such a smooth surface can facilitate analysis of the biological material 108 in the well-like depression(s) 128 via microscopy.

It is noted that such a smooth surface, e.g. achieved via the high-gloss polished surface of the mold, can be included in the biocompatible support portion 106 of the support member(s) 104 of any of the embodiments described herein.

In some embodiments, a base of the, e.g. each of the, well-like depression(s) 128 delimits the apertures 126.

In such embodiments, fluid may be able to pass through the apertures 126 to reach the biological material 108 located in the well-like depression(s) 128.

The apertures 126 delimited by the base of the well-like depression(s) 128 may each have a diameter, or if not circular a largest dimension across the respective aperture 126, of less than about 10 µm.

In some embodiments, the apertures 126 may be formed in the base of the well-like depression(s) 128, for example, by laser ablation. For instance, the apertures 126 may be formed throughout an area of a membrane, e.g. a 20 µm thickness membrane, whereas a main part of the support member 104 has only the well-like depression(s) 128 defined therein. The main part may be thicker, e.g. 0.5 mm thick, than the membrane that defines the base of the well-like depression(s) 128.

This may represent a more efficient way of attaining the apertures-comprising base of the well-like depression(s) 128 compared to, for example, laser ablating the base of each individual well-like depression 128. If several well-like depressions 128 are included in the biocompatible support portion 106, the latter may require interruption of the laser ablation process and repositioning/reorientating the laser ablation set-up when switching from ablating the base of one well-like depression 128 to ablating the base of another well-like depression 128.

In some embodiments, several of the depression(s)-comprising biocompatible support portions 106 can be molded, e.g. injection molded, in a slab of material, e.g. the elastomeric material, and each of the biocompatible support portions 106 subsequently punched out of the slab. Alternatively, the biocompatible support portion 106 can be injection molded directly, without being punched out of such a slab.

In some embodiments, the biocompatible membrane, e.g. the perforate biocompatible membrane, may, for instance, be included in the support member 104 together with the well-like depression(s)-comprising biocompatible support portion 106. For example, the perforate biocompatible membrane may be arranged in the substrate 102 beneath the biocompatible support portion 106 in which the well-like depression(s) is/are defined.

In some embodiments, a first support member 104 whose biocompatible support portion 106 has the well-like depression(s) 128 can be stacked in the substrate 102 with a second support member 104 having the biocompatible membrane, e.g. the perforate biocompatible membrane, so that the biological material 108 is retainable between the depression(s)-comprising biocompatible support portion 106 and the biocompatible membrane. In such embodiments, the biological material 108 may, for example, comprise spheroids or organoids.

In some embodiments, the well-like depression(s) 128 can be relatively deep, for example with a depth of about 1 mm, so that the biocompatible membrane can cover the well-like depression(s) 128.

In this manner, the spheroids or organoids cannot, for example, migrate into other well-like depressions 128, or risk being released and flushed away with a flow in a fluidic device.

In other words, the stacked depression(s)-comprising biocompatible support member 106 and biocompatible membrane may define "micro box(es)" in which the formed organoids or spheroids can be retained (i.e. "boxed in").

In some embodiments, a first support member 104 whose biocompatible support portion 106 has the apertures 126 can be stacked in the substrate 102 with a second support member 104 having the biocompatible membrane, e.g. the perforate biocompatible membrane, so that the biological material 108 is retainable between the biocompatible support portion 106 and the biocompatible membrane. In such embodiments, the biological material 108 may, for example, comprise tissue, cell layers or biopsies.

The multiple, stacked support members 104 described above can, for example, be arranged in one of the chamber unit(s) 110 defined in the substrate 102.

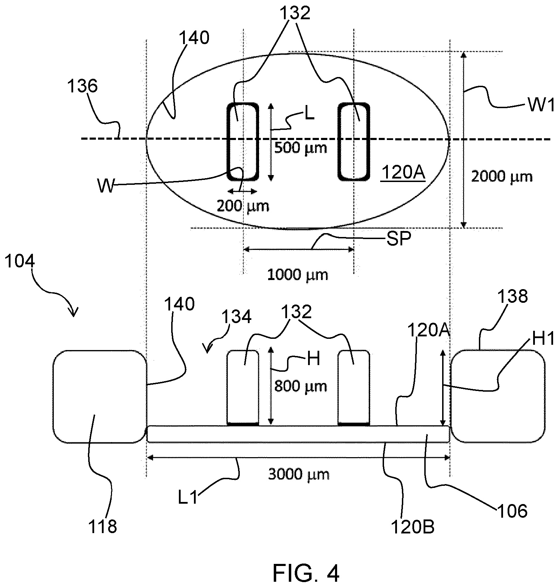

In some embodiments, such as shown in FIG. 4, the biocompatible support portion 106 comprises a pair of flexible protrusions 132 for supporting tissue thereon and therebetween. In such embodiments, the system 100 may be employed to test muscle tissue, e.g. cardiac tissue, grown between and around the pair of protrusions 132. When the muscle tissue contracts, the pair of protrusions 132 may deflect or bend towards each other, with displacement of the protrusions 132 caused by the deflection being measurable to enable determination of one or more contraction properties such as for example an extent of contraction, a frequency of contraction and/or a force of contraction provided by contraction of the tissue.

The protrusions may have any suitable height H provided that they are able to fulfil their tissue testing function. In some embodiments, the protrusions 132 each have a height H or largest dimension that is 500 to 1500 µm, preferably 600 to 1000 µm.

Such a height H, e.g. together with the thickness of the biocompatible support portion 106 between the surface 120B to the surface 120A from which the protrusions 132 protrude, being at most 4 mm, may assist with analysis of the tissue by optical microscopy through the biocompatible support portion 106 when the biocompatible support portion 106 is optically transparent at least beneath the protrusions 132.

Alternatively, or additionally, the protrusions 132 may each have a width W of 100 to 300 µm and/or a length L of 400 to 600 µm.

A spacing SP between the protrusions 132, e.g. protrusions 132 having the above height H, width W and/or length L dimensions, may be 800 to 1200 µm.

It is noted that the flexibility of the protrusions 132 may be provided by at least the protrusions of the biocompatible support portion 106 comprising an elastomeric material, such as an elastomeric material comprising one or more materials chosen from the group consisting of silicone and polybutadiene.

In some embodiments, such as shown in FIG. 4, a recess 134 is defined in the support member 104. A base surface 120A that partly delimits the recess 134 may be joined to a main surface 138 of the support member 104 by a side surface 140 that extends between the base surface 120A and the main surface 138.

The flexible protrusions 132 may be arranged on and/or in the base surface 120A of the recess 134. Alternatively, or additionally, the recess 134 may be elongated, e.g. ellipsoidal, when viewed in plan, as shown in the upper pane of FIG. 4.

In the case of such an elongated recess 134, and when the protrusions 132 are included in the support member 104, the protrusions 132 may be arranged along a longitudinal axis 136 along which the recess 134 is elongated. Contraction of muscle tissue between the protrusions 132 may be along this longitudinal axis 136.

The recess 134, e.g. elongated recess 134, can have any suitable dimensions. In some embodiments, and referring to FIG. 4, the recess 134 has a width W1 of 1500 to 2500 µm, preferably 1800 to 2200 µm, and/or a length L1 of 2500 to 3500 µm, preferably 2700 to 3200 µm. Plainly the length L1, extending along the longitudinal axis 136, is larger than the width W1 in embodiments in which the recess 134 is elongated.

The height H1 of the recess 134, e.g. the recess 134 having the above width W1 and/or length L1 dimensions, may be 500 to 1500 µm, preferably 600 to 1000 µm.

It is noted that the height H1 of the recess 134 may be the same, or within 10%, of the height H of the protrusions 132.

It is noted that in some embodiments, the protrusions 132 are included in the biocompatible support portion 106, but without any recess 134 being defined in the support member 104.

In such embodiments, the sidewall(s) of the chamber unit(s) 110 may, for example, be relied upon to provide suitable lateral confinement of the biological material 108, cell culture medium, etc. within each of the chamber unit(s) 110.

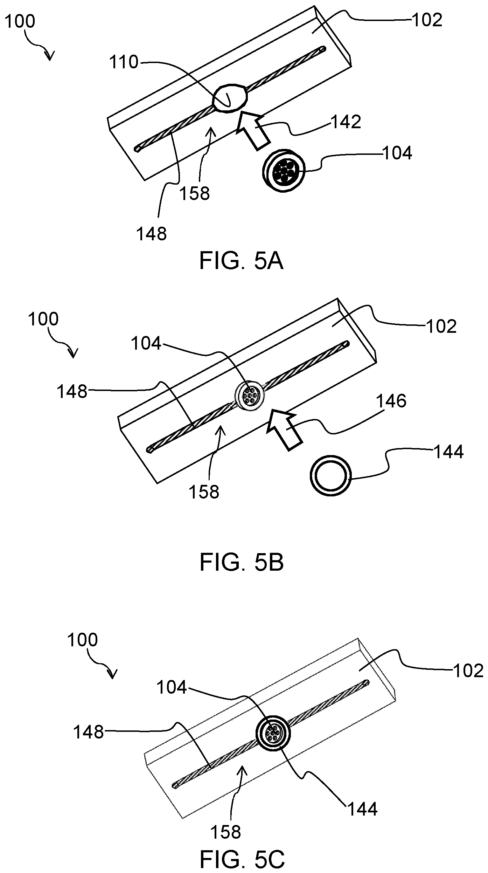

FIGs. 5A to 5C show mounting of a support member 104 to a substrate 102 according to a non-limiting illustrative example. The support member 104 may be inserted into a chamber unit 110 defined in the substrate 102 (see the arrow 142 in FIG. 5A), and once inserted the support member 104 may be secured in place using an attachment member 144, e.g. attachment ring (see the arrow 146 in FIG. 5B denoting securing of the support member 104 to the substrate 102 using the attachment member 144). The detachment process may be the reverse of the mounting process: removal of the attachment member 144, followed by withdrawal of the support member 104 from the chamber unit 110, e.g. via the manual manipulation.

It is noted that the attachment member 144, e.g. attachment ring, can, for example, be made of metal, such as stainless steel. A stainless steel attachment member 144 was found not to disturb oncology testing using the system 100 shown in FIGs. 5A to 5C.

In some embodiments, such as shown in FIGs. 5A to 5C, one or more fluidic channels 148 is/are defined in the substrate 102 for supplying fluid, e.g. cell culturing fluid, to and/or removing fluid from the biological material 108 supported by the support member 104.

In some embodiments, fluidic path(s) may be defined in the manipulation portion 118, e.g. in the rim member or ledge member, which fluidic path(s) permit(s) fluid to pass from the fluidic channel(s) 148 through the manipulation portion 118 to reach the biocompatible support portion 108.

Such fluidic path(s) may be useful particularly in embodiments in which the support member 104 is placeable at the bottom of the chamber unit 110, rather than being supported by the supporting region(s) 114.

It is noted that when mounting the support member 104 to the substrate 102, the fluidic path(s) may be aligned with the fluidic channel(s) 148, e.g. with the assistance of the orientation indicator system 122, 124.

Whilst only one chamber unit 110 is defined in the substrate 102 shown in FIGs. 5A to 5C, this should not be regarded as being limiting, and in other embodiments, such as shown in FIG. 6, a plurality of chamber units 110 are defined in the substrate 102.

It is noted that such a plurality of chamber units 110 may be arranged in the substrate 102 in an ordered manner, for example in a rectangular array. Alternatively, the chamber units 110 may be distributed randomly in the substrate 102 (not shown).

It is further noted that the substrate 102 shown in FIG. 6 can be regarded as an example of a standardized 96 well/chamber unit 110 cell plate (e.g. with the wells having a diameter of about 6 mm). An advantage of standardized substrates 102 is that the system 100 can be straightforwardly used in conjunction with existing optical inspection devices, e.g. microscopes, for assessing the biological material 108.

Using, for example, a hand tool, such as tweezers or forceps, the support member(s) 104 may be inserted into one or more of the chamber units 110 of the substrate shown in FIG. 6.

The arrow 150 in FIG. 6 is for denoting mounting of the support member 104 to the substrate 102 via insertion of at least part of the support member 104 into one of the chamber units 110.

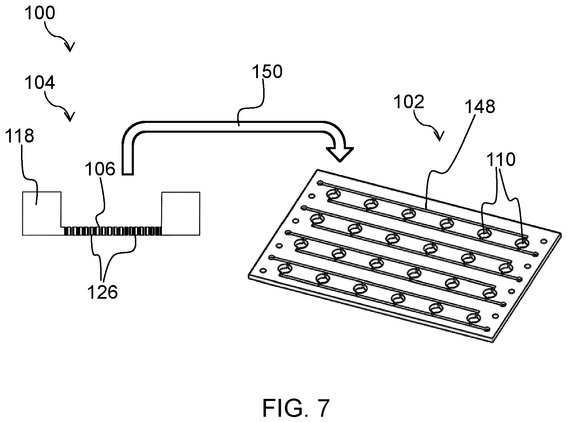

In some embodiments, such as shown in FIG. 6, adding of fluid to and removing of fluid from the chamber unit(s) 110 may be implemented using a pipette arrangement. Alternatively or additionally, and referring now to FIGs. 7 and 8, fluidic channel(s) 148 may be provided for adding fluid to and/or removing of fluid from the chamber unit(s) 110.

FIGs. 7 and 8 show a substrate 102 in the form of a fluidic device having, in this non-limiting example, twenty four chamber units 110. The support member(s) 104 can be mounted to this substrate 102 by inserting the support member(s) 104 into one or more of the twenty four chamber units 110, e.g. using a hand tool, such as tweezers or forceps.

The substrate 102 shown in FIGs. 7 and 8 has a top side 154 and a bottom side 156 opposite the top side 154. The substrate 102 may comprise one or more fluidic assemblies 158, and in the case of the embodiment shown in FIGs. 7 and 8, the substrate 102 comprises four fluidic assemblies 158. The number of fluidic assemblies 158 included in the substrate 102 can vary according to testing requirements, and in other embodiments the substrate 102 comprises a single fluidic assembly 158 (see FIGs. 5A to 5C), or two, three, five, six, seven, eight or more fluidic assemblies 158.

Each, or in other cases at least one, of the one or more fluidic assemblies 158 may comprise at least one chamber unit 110 in which the biological material 108 is receivable. The at least one chamber unit 110 defined in the substrate 102 may extend between the top side 154 and the bottom side 156.

The at least one chamber unit 110 may extend to an upper opening delimited by the top side 154 of the substrate 102 and/or may extend to a lower opening delimited by the bottom side 156 of the substrate 102. In such embodiments, fluid retention in and/or contamination protection of the at least one chamber unit 110 can be assisted by the system 100 including an upper cover (not visible) for covering the top side 154 of the substrate 102 and/or a lower cover (not visible) for covering the bottom side 156 of the substrate 102. Regarding fluid retention, such an upper cover and/or lower cover can assist to minimize loss of fluid via e.g. evaporation.

In some embodiments, one or both of the upper and lower covers is or are configured to enable access to the at least one chamber unit 110, for example for fluid delivery into and/or fluid withdrawal from the at least one chamber unit 110, e.g. via a pipette arrangement.

For example, one or both of the upper and lower covers may be releasably mounted to the substrate 102, with release of the respective cover enabling access to the at least one chamber unit 110.

The upper and lower covers can be formed of any suitable material. Particular mention is made of optically transmissive materials, such as glass and optically transmissive polymers, such as polycarbonate. This is because such materials may facilitate monitoring of testing taking place in the system 100, for instance via optical microscopy.

The upper and lower covers can be mounted, e.g. releasably mounted, to the substrate 102 in any suitable manner, for example using adhesive tape, clips, and so on. A non-limiting example of suitable adhesive tape is Avery Dennison MED 1125.

Preferably, when the one or more upper and lower covers are mounted, the adhesion is such that the fluid can be manipulated with (slightly) elevated pressure using e.g. an external pumping system or for example a flow control system, as previously mentioned.

The fluidic assembly 158 may include an inlet 160 at which fluid, for example fluid containing cell nourishment and/or a drug formulation, is deliverable to the substrate 102, and an outlet 162 at which fluid is permitted to leave the substrate 102, for example for the purpose of carrying waste away from the chamber unit(s) 110 and/or to enable replenishing of the cell nourishment and/or drug formulation.

It is reiterated that a flow control system (not visible) can be used to supply the fluid to the inlet 160 and remove fluid via the outlet 162.

As shown in FIG. 8, an inlet fluidic channel 148A may be defined in the substrate 102, with the inlet fluidic channel 148A extending between the inlet 160 and the at least one chamber unit 110, e.g. the upper portion of the chamber unit(s) 110, while an outlet fluid channel 148B is defined in the substrate 102 and extends between the at least one chamber unit 110, e.g. the upper portion thereof, and the outlet 162. Thus, the inlet fluid channel 148A and the outlet fluid channel 148B may fluidly connect the inlet 160 and the outlet 162 to the chamber unit(s) 110.

In some embodiments, at least one lower fluidic channel may, for instance, extend between a lower fluid inlet 164 and a lower fluid outlet 166 via the chamber unit(s) 110, e.g. via the lower portion of the chamber unit(s) 110. The flow control system may be connectable via tubing to the lower fluid inlet 164 and the lower fluid outlet 166.

One of the inlet fluidic channel 148A and the lower fluid channel may, for example provide nutrition to the biological material 108 and the other of the inlet fluidic channel 148A and the lower fluid channel may, for instance, provide a drug to be tested to the biological material 108.

In some embodiments, such as shown in FIG. 9, a dual chamber unit 110 is included in one or more of the at least one chamber unit 110, with the dual chamber unit 110 comprising a first chamber 168 and a second chamber 170 fluidly connected to each other via a bridge defined between the first chamber 168 and the second chamber 170. In such embodiments, the at least part of the at least one support member 104 may be insertable into the first chamber 168 and/or the second chamber 170.

Such a dual chamber unit 110 may have utility in studying metastasis of cancerous cells. It may be possible to assess cells that are released or emitted by any cell cultures that are provided in the first chamber 168. The first chamber 168 can therefore be used to perform a treatment on the cell culture(s), such as the application of a certain therapeutic fluid or drug, for example a chemotherapy treatment fluid, with the second chamber 170 capturing any released cells to analyze the effect of the treatment performed in the first chamber 168 on metastasis.

Whilst the underside 156 of the substrate 102 is not shown in detail in the view provided in FIG. 9, it is noted that the lower fluidic channel(s) described above in relation to the embodiment shown in FIGs. 7 and 8 may be included in the substrate 102 shown in FIG. 9.

More generally, the present disclosure proposes use of the system 100 according to any of the embodiments described herein for testing biological material, for example for testing cell groups, double cell layers, spheroids, organoids, biopsies, or combinations thereof.

The detachable support member(s) 104 according to the present disclosure may, for example, be used to manipulate cells present on the biocompatible support portion 106, e.g. on the biocompatible membrane.

The present disclosure further contemplates a method of testing biological material comprising: supporting the biological material on the biocompatible support portion 106 included in the at least one support member 104 of the system 100 according to any of the embodiments described herein, with the at least one support member 104 being mounted to the substrate 102; and manually manipulating the at least one support member 104 to detach the at least one support member 104 from the substrate 102.

For example, after pretreatment in a chamber unit 110 of a standard well cell plate-type substrate 102 to attach and grow initial cells, the support member 104 can be detached from the substrate 102 and transferred to a further substrate, e.g. fluidic device(s), for instance for oncology tests.

In more general terms, the system 100 may include the substrate 102 and a further substrate to which the support member(s) 104 is/are mountable.

In such embodiments, the support member(s) 104 may also be detachable from the further substrate, for instance at least by manual manipulation by the user.

In some embodiments, the method comprises exposing the biological material 108 to a drug to be tested.

In such embodiments, the method may further comprise monitoring a response of the biological material 108, e.g. cultured cells, to the drug to be tested. This monitoring may be implemented in any suitable manner, for example by optical microscopy.

Here a protocol is described, by way of non-limiting illustrative example, which demonstrates that cells can be transferred between different substrates/devices. The following protocol is provided as an illustrative non-limiting example of application of a system 100 according to the present disclosure:
- A substrate 102 in the form of a 24 well plate is provided.
- 0.25 ml gelatine (2 % w/v) is added to a chamber unit 110/well of the substrate 102.
- A support member 104 comprising a biocompatible silicone membrane as the biocompatible support portion 106 is placed in the chamber unit 110.
- After overnight coating, the support member 104 is detached from the substrate 102 by being withdrawn from the chamber unit 110 using tweezers, and placed in an empty adjacent chamber unit 110.
- Cell suspension is added onto the biocompatible silicone membrane.
- The support member 104 is withdrawn and inverted after overnight incubation and placed in another empty chamber unit 110 (see, e.g., FIGs. 1E and 1F).
- Another cell suspension is added onto the inverted biocompatible silicone membrane for co-culture (see, e.g., FIG. 1G).
- After overnight incubation, the support member 104 is removed from the substrate 102 using tweezers and placed in a chip holder of a fluidic device.
- The fluidic device is closed and used in a fluidic flow experiment.

Upon completion of the experiment, the support member(s) 104 can be removed from the setup and further analysis, such as a staining, can be performed.

The following exemplary commercially available elastomeric/soft materials (which are transparent or translucent and are food contact approved) can be used, in certain embodiments, as the material for the support member 104, e.g. the biocompatible support portion 106 thereof: Medalist MD-53253 (TPE Teknor Apex); Medalist MD-53273 (TPE Teknor Apex); Mediprene 500602 M-03 (TPE Hexpol); Texin Rx T85A (TPU Covestro); BioSpan^{®} (F SPU | PUR); BioSpan^{®} (SPU | PUR); BJB Polyurethane F-116 A/B | TSU; BJB Polyurethane F-126 A/B | TSU; BJB Polyurethane F-131 A/B | TSU; BJB Polyurethane M-3115 REV 1 A/B | TSU; BJB Polyurethane M-3125 A/B | TSU; CELLENE MC2248 | TPE; CELLENE MC2265 | TPE; CELLENE MC3038 | TPE; CELLENE MC3050 | TPE; CELLENE MC3061 | TPE; CELLENE MC3226 | TPE; CELLENE MC3239 | TPE; CELLENE MC3261 | TPE; Dynaflex^{™} G2706-1000-00 | TPE; Dynaflex^{™} G2711-1000-00 | TPE; Elastocon^{®} 2860L | TPE; Filter-bond^{™} E-3264 | TS; FLEXCHEM^{™} 3551-02 | PVC, Flexible; FLEXCHEM^{™} 4051-02 | PVC, Flexible; FLEXCHEM^{™} 4551-02 | PVC, Flexible; FLEXCHEM^{™} 5051-02 | PVC, Flexible; FLEXCHEM^{™} 5551-02 | PVC, Flexible; FLEXCHEM^{™} 6051-02 | PVC, Flexible; FLEXCHEM^{™} 6551-02 | PVC, Flexible; Medalist^{®} MD-12130 | TPE; Medalist^{®} MD-12130H | TPE; Medalist^{®} MD-12140 | TPE; Medalist^{®} MD-12140H | TPE; Medalist^{®} MD-12150 | TPE; Medalist^{®} MD-12150H | TPE; Medalist^{®} MD-12f150S | TPE; Medalist^{®} MD-12160 | TPE; Medalist^{®} MD-12160H | TPE; Medalist^{®} MD-12170 | TPE; Medalist^{®} MD-12170H | TPE; Medalist^{®} MD-12243 | TPE; Medalist^{®} MD-12337 | TPE; Medalist^{®} MD-12340 NAT | TPE; Medalist^{®} MD-12342 | TPE; Medalist^{®} MD-12344 | TPE; Medalist^{®} MD-12350 | TPE; Medalist^{®} MD-12352 | TPE; Medalist^{®} MD-12362 | TPE; Medalist^{®} MD-125 | TPE; Medalist^{®} MD-130 | TPE; Medalist^{®} MD-13240 | TPE; Medalist^{®} MD-135 | TPE; Medalist^{®} MD-145 | TPE; Medalist^{®} MD-155 | TPE; Medalist^{®} MD-17365 | TPE; Medalist^{®} MD-225 | TPV; Medalist^{®} MD-32045 | TPE; Medalist^{®} MD-32245 | TPE; Medalist^{®} MD-36048 | TPE; Medalist^{®} MD-37063 NAT | TPE; Medalist^{®} MD-42245 XRD1 | TPE; Medalist^{®} MD-42245 XRD3 | TPE; Medalist^{®} MD-74357 XRD1 | TPE; Medalist^{®} MD-74357 XRD2 | TPE; Mediprene^{®} 500120M | TPE; Mediprene^{®} 500200M | TPE; Mediprene^{®} 500250M | TPE; Mediprene^{®} 500300M | TPE; Mediprene^{®} 500350M | TPE; Mediprene^{®} 500400M | TPE; Mediprene^{®} 500434M | TPE; Mediprene^{®} 500450M | TPE; Mediprene^{®} 500484M | TPE; Mediprene^{®} 500520M | TPE; Mediprene^{®} 500534M | TPE; Mediprene^{®} 500584M | TPE; Mediprene^{®} 500600M | TPE; Mediprene^{®} 500634M | TPE; Mediprene^{®} 500650M | TPE; Mediprene^{®} 500684M | TPE; Mediprene^{®} 500700M | TPE; Monprene^{®} RG-10160H | TPE; ProvaMed^{®} TPE 1120 | TPE; ProvaMed^{®} TPE 1160 | TPE; RABALON^{®} PJ4300C | TPE; RABALON^{®} PJ5300C | TPE; RABALON^{®} PJ6300C | TPE; RABALON^{®} PJ7300C | TPE; SkinFlex 15 F-115 A/B | TSU; SkinFlex BR-60; BRUSHABLE A/B | TSU; T-Blend^{®} TPE-F22 | SEBS; THERMOLAST^{®} M TM3LFT (Series: MC/LF) | TPE; THERMOLAST^{®} M TM3MED (Series: MC/tl) | TPE; THERMOLAST^{®} M TM3RST (Series: MC/RS) | TPE; THERMOLAST^{®} M TM4LFT (Series: MC/LF) | TPE; THERMOLAST^{®} M TM4MED (Series: MC/tl) | TPE; THERMOLAST^{®} M TM4RST (Series: MC/RS) | TPE; THERMOLAST^{®} M TM5LFT (Series: MC/LF) | TPE; THERMOLAST^{®} M TM5MED (Series: MC/tl) | TPE; THERMOLAST^{®} M TM6LFT (Series: MC/LF) | TPE; THERMOLAST^{®} M TM6MED (Series: MC/tl) | TPE; THERMOLAST^{®} M TM7LFT (Series: MC/LF) | TPE THERMOLAST^{®} M TM7MED (Series: MC/tl) | TPE; UNISOFT SPECIAL^{™} DS-35A-CL-M-01 | SEBS; UNISOFT SPECIAL^{™} DS-55A-CL-M-01 | SEBS; Versaflex^{™} G2705 N | TPE; Versaflex^{™} HC 1100-40 Translucent EU | TPE; Versaflex^{™} HC 1348 Natural | TPE; Versaflex^{™} HC MT317 | TPE; Versaflex^{™} HC MT555 | TPE; Versaflex^{™} OM 1040X-1 | TPE; CELLENE MC2248 | TPE; CELLENE MC2265 | TPE; CELLENE MC3038 | TPE; CELLENE MC3050 | TPE; CELLENE MC3061 | TPE; CELLENE MC3226 | TPE; CELLENE MC3239 | TPE; CELLENE MC3261 | TPE; ChronoPrene^{™} 25A | TPE; ChronoPrene^{™} 40A | TPE (CardioTech International, Inc.); Dryflex^{®} 500300S | TPE; Dryflex^{®} 500350S | TPE; Dryflex^{®} 500400S | TPE; Dryflex^{®} 500450S | TPE; Dryflex^{®} 500500S | TPE; Dryflex^{®} 500550S | TPE; Dryflex^{®} 500600S | TPE; Dryflex^{®} 500650S | TPE; Dryflex^{®} 500700S | TPE; Dynaflex^{™} G2701-1000-02 | TPE; Dynaflex^{™} G2706-1000-00 | TPE; Dynaflex^{™} G2709-1000-00 | TPE; Dynaflex^{™} G2711-1000-00 | TPE; Dynaflex^{™} G2712-1000-02 | TPE; Dynaflex^{™} G2730 | TPE; Dynaflex^{™} G2755-1000-00 | TPE; Dynaflex^{™} G2755C | TPE; Dynaflex^{™} G6713-0001 | TPE; Dynaflex^{™} G6713C | TPE; Dynalloy^{™} GP 7810-60T | TPE; Dynalloy^{™} GP 7810-70T | TPE; Dynalloy^{™} OBC8200-BT50 | TPE; Estane^{®} 58123 TPU | TPU-Polyether; Evoprene^{™} 019 | SBS; Evoprene^{™} G 925 | SEBS; Evoprene^{™} G 936 | SEBS; Evoprene^{™} G 942 | SEBS; Evoprene^{™} G 958 | SEBS; Evoprene^{™} G 966 | SEBS; Evoprene^{™} G 967 | SEBS; Evoprene^{™} G 968 | SEBS; Evoprene^{™} G 969 | SEBS; Evoprene^{™} G 970 | SEBS; Evoprene^{™} GC 5685 | SEBS; Evoprene^{™} GC 5686 | SEBS; Evoprene^{™} GC 5687 | SEBS; Evoprene^{™} GC 5688 | SEBS; Evoprene^{™} GC 5689 | SEBS; Evoprene^{™} GC 5690 | SEBS; GLS 422-126 | TPE; GLS 458-140 | TPE; GLS 458-141 | TPE; GLS 458-142 | TPE; K-Prene HYFLEX HF 15 | MPR; K-Prene HYFLEX HF 20 | MPR; K-Prene HYFLEX HF 25 | MPR; K-Prene HYFLEX HF 30 | MPR; Medalist^{®} RG-38052 XRD1 | TPE; megol^{®} PUG 10 | SEBS; megol^{®} PUG 60 | SEBS; megol^{®} TA 60 | SEBS; Monprene^{®} RG-10130 | TPE; Monprene^{®} RG-10140 | TPE; Monprene^{®} RG-10150 | TPE; Monprene^{®} RG-10160 | TPE; Monprene^{®} RG-10170 | TPE; Monprene^{®} RG-15130 | TPE; Monprene^{®} RG-15140 | TPE; Monprene^{®} RG-15150 | TPE; Monprene^{®} RG-15160 | TPE; Monprene^{®} RG-15170 | TPE; Monprene^{®} RG-18240 | TPE; Monprene^{®} RG-18250 | TPE; Monprene^{®} RG-18260 | TPE; Monprene^{®} RG-18270 | TPE; Monprene^{®} RG-19221 NAT | TPE; Monprene^{®} RG-19255 | TPE; Monprene^{®} RG-20140 | TPE; Monprene^{®} RG-20160 | TPE; Monprene^{®} RG-20170 | TPE; Monprene^{®} RG-29068 NAT | TPE; Monprene^{®} RG-29240 XRD1 | TPE; RABALON^{®} MJ4300C | TPE; RABALON^{®} MJ5302C | TPE; RABALON^{®} MJ6301C | TPE; RABALON^{®} MJ7301C | TPE; RAYPRENE^{®} NB221-S4050 | TPE; RAYPRENE^{®} NB221-S4051 | TPE; RAYPRENE^{®} NB221-S4052 | TPE; RAYPRENE^{®} NB221-S4053 | TPE; tefabloc^{®} TO 132 | TPE; Telcar^{®} TL-83-F943D22-NT BLU | TPE; THERMOLAST^{®} K TF2CGT (Series: FC) | TPE; THERMOLAST^{®} K TF3BTL (Series: FC/AP) | TPE; THERMOLAST^{®} K TF3CGT (Series: FC) | TPE; THERMOLAST^{®} K TF3STE (Series: FC/CS) | TPE; THERMOLAST^{®} K TF4AAB (Series: FC/HE/tl) | TPE; THERMOLAST^{®} K TF4BTL (Series: FC/AP) | TPE; THERMOLAST^{®} K TF4CGT (Series: FC) | TPE; THERMOLAST^{®} K TF4STE (Series: FC/CS) | TPE; THERMOLAST^{®} K TF5AAC (Series: FC/HE/tl) | TPE; THERMOLAST^{®} K TF5BTL (Series: FC/AP) | TPE; THERMOLAST^{®} K TF5CGT (Series: FC) | TPE; THERMOLAST^{®} K TF5STE (Series: FC/CS) | TPE; THERMOLAST^{®} K TF5WHA (Series: DW/H) | TPE; THERMOLAST^{®} K TF6AAF (Series: FC/HE/tl) | TPE; THERMOLAST^{®} K TF6BTL (Series: FC/AP) | TPE; THERMOLAST^{®} K TF6CGT (Series: FC) | TPE; THERMOLAST^{®} K TF6STE (Series: FC/CS) | TPE; THERMOLAST^{®} K TF6WCS (Series: DW/CS) | TPE; THERMOLAST^{®} K TF6WHA (Series: DW/H) | TPE; THERMOLAST^{®} K TF6WHB (Series: DW/H) | TPE; THERMOLAST^{®} K TF7AAC (Series: FC/HE/tl) | TPE; THERMOLAST^{®} K TF7BTL (Series: FC/AP) | TPE; THERMOLAST^{®} K TF7CGT (Series: FC) | TPE; THERMOLAST^{®} K TF7WHB (Series: DW/H) | TPE; Topolymer^{®} 8201-B | TPE; Versaflex^{™} FFC 2882-50 EU | TPE; Versaflex^{™} FFC 2882-50 | TPE; Versaflex^{™} G2708 N | TPE; Versaflex^{™} GP 2810-20N | TPE; Versaflex^{™} GP 2810-30N | TPE; Versaflex^{™} GP 2810-40N | TPE; Versaflex^{™} GP 2810-50N | TPE; Versaflex^{™} GP 2810-60N | TPE; Versaflex^{™} GP 2810-70N | TPE; Cawiton^{®} MT920 | SEBS; Cawiton^{®} MT930 | SEBS; Cawiton^{®} MT940 | SEBS; Cawiton^{®} MT950 | SEBS; Cawiton^{®} MT960 | SEBS; Cawiton^{®} MT970 | SEBS.

The following embodiments are also disclosed:
Embodiment 1. A system (100) for use in testing biological material (108), the system comprising:
   a substrate (102); and
   at least one support member (104) comprising a biocompatible support portion (106) for supporting the biological material, the at least one support member being mountable to, and detachable from, the substrate, wherein the at least one support member is detachable from the substrate at least by a user, preferably wherein the detachability is by manual manipulation by the user.
Embodiment 2. The system (100) according to Embodiment 1, wherein the at least one support member (104) is configured so that the biological material (108) remains supported by the biocompatible support portion (106) during the detachment of the at least one support member from the substrate (102).
Embodiment 3. The system (100) according to Embodiment 1 or Embodiment 2, wherein the at least one support member (104) comprises a manipulation portion (118) that is manipulable to permit the detachment of the at least one support member from the substrate (102), the manipulation portion being arranged adjacent to the biocompatible support portion (106); optionally wherein the manipulation portion (118) comprises a rim member or ledge member arranged around at least part of a periphery of the biocompatible support portion (106).
Embodiment 4. The system (100) according to any one of Embodiments 1 to 3, comprising an orientation indicator system (122, 124) configured to guide mounting of each of the at least one support member (104) in a defined reference orientation relative to the substrate (102); optionally wherein the orientation indicator system (122, 124) comprises a shape feature (122) of the each of the at least one support member (104) and a further shape feature (124) of the substrate (102), the shape feature and the further shape feature fitting to each other upon adoption of the defined reference orientation.
Embodiment 5. The system (100) according to any one of Embodiments 1 to 4, wherein the biocompatible support portion (106) comprises a first surface (120A) and a second surface (120B) on which the biological material (108) is supportable, and wherein the system is switchable between a first configuration and a second configuration, wherein in the first configuration the at least one support member (104) is mounted to the substrate (102) so that one of the first and second surfaces (120A, 120B) is facing in a given direction, the at least one support member being detachable from the substrate and re-mountable to the substrate so that the other of the first and second surfaces (120B, 120A) is facing in the given direction in the second configuration.
Embodiment 6. The system (100) according to any one of Embodiments 1 to 5, wherein the biocompatible support portion (106) of one or more of the support member(s) (104) comprises a biocompatible membrane on which the biological material (108) is supportable.
Embodiment 7. The system (100) according to any one of Embodiments 1 to 6, wherein the biocompatible support portion (106) of one or more of the support member(s) (104) delimits apertures (126) for allowing fluid to pass therethrough to reach the biological material (108) supported by the biocompatible support portion; optionally wherein each of the apertures (126) has a diameter, or if not circular a largest dimension across the respective aperture, of less than about 10 µm.
Embodiment 8. The system (100) according to any one of Embodiments 1 to 7, wherein well-like depression(s) (128) is/are defined in a biological material-contacting surface (130) of the biocompatible support portion (106) of one or more of the support member(s) (104); and/or wherein the biocompatible support portion (106) of one or more of the support member(s) (104) comprises a pair of flexible protrusions (132) for supporting tissue thereon and therebetween.
Embodiment 9. The system (100) according to any one of Embodiments 1 to 8, wherein at least one chamber unit (110) is defined in the substrate (102), wherein the at least one support member (104) is mountable to the substrate via insertion of at least part of the at least one support member into one of the at least one chamber unit, and wherein the at least one support member is detachable, at least by said manual manipulation, from the substrate via withdrawal of the at least part of the at least one support member from said one of the at least one chamber unit; optionally wherein the biocompatible support portion (106) separates an upper portion from a lower portion of the respective chamber unit (110) when the at least part of the at least one support member (104) is inserted into said one of the at least one chamber unit.
Embodiment 10. The system (100) according to Embodiment 9, wherein the at least one chamber unit (110) comprises a plurality of chamber units defined in the substrate (102).
Embodiment 11. The system (100) according to any one of Embodiments 1 to 10, wherein the at least one support member (104) comprises a plurality of support members; optionally wherein two or more support members (104) of the plurality of support members are mountable to the substrate (102) by receiving at least part of each of the two or more support members in a chamber unit (110) defined in the substrate.
Embodiment 12. The system (100) according to any one of Embodiments 1 to 11, wherein the biocompatible support portion (106) comprises an elastomeric material that provides flexibility to the biocompatible support portion; and/or wherein the biocompatible support portion (106) comprises a cell culture protein for contacting the biological material (108).
Embodiment 13. Use of the system (100) according to any one of Embodiments 1 to 12 for testing biological material (108).
Embodiment 14. A method of testing biological material (108), the method comprising:
   supporting the biological material on the biocompatible support portion (106) included in the at least one support member (104) of the system (100) according to any one of claims 1 to 12, the at least one support member being mounted to the substrate (102); optionally wherein said supporting biological material comprises culturing cells on the biocompatible support portion; and
   manually manipulating the at least one support member to detach the at least one support member from the substrate.
Embodiment 15. A support member (104) for supporting biological material (108), the support member being mountable to, and detachable from, a substrate (102), the support member comprising a biocompatible support portion (106) for supporting the biological material.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A support member (104) for use with a system for testing biological material, the system comprising a substrate and the support member comprising a biocompatible support portion (106) for supporting the biological material, the support member being mountable to, and detachable from, the substrate, wherein the support member is detachable from the substrate by a user.

2. The support member claim 1, wherein the support member (104) is configured so that the biological material (108) remains supported by the biocompatible support portion (106) during the detachment of the at least one support member from the substrate (102).

3. The support member according to claim 1 or 2, wherein the support member (104) comprises a manipulation portion (118) that is manipulable to permit the detachment of the at least one support member from the substrate (102), the manipulation portion being arranged adjacent to the biocompatible support portion (106).

4. The support member according to claim 3, wherein the manipulation portion (118) comprises a rim member or ledge member arranged around at least part of a periphery of the biocompatible support portion (106).

5. The support member according to any one of claims 1 to 4, the support member comprising a first orientation feature of an orientation indicator system (122, 124) the first feature configured to cooperate with a second orientation feature of the orientation indicator system such that the cooperation is capable of guiding mounting of the support member (104) in a defined reference orientation relative to the substrate (102).

6. The support member according to claim 5, wherein the first orientation feature comprises a shape feature (122)) and the second orientation feature comprises a further shape feature (124) and the further shape feature is part of the substrate (102), the shape feature and the further shape feature fitting to each other upon adoption of the defined reference orientation.

7. The support member according to any one of Claims 1 to 6, wherein the biocompatible support portion (106) comprises a first surface (120A) on which the biological material (108) is supportable, and a second surface (120B) on which the biological material (108) is supportable, wherein in a first configuration the support member (104) is mountable to the substrate (102) so that one of the first and second surfaces (120A, 120B) is facing in a given direction, the at least one support member being detachable from the substrate and re-mountable to the substrate so that the other of the first and second surfaces (120B, 120A) is facing in the given direction in the second configuration.

8. The support member according to any one of Claims 1 to 7, wherein the biocompatible support portion (106) of the support member (104) comprises a biocompatible membrane on which the biological material (108) is supportable.

9. The support member according to any one of claims 1 to 8, wherein the biocompatible support portion (106) of the support member (104) delimits apertures (126) for allowing fluid to pass therethrough to reach the biological material (108) supported by the biocompatible support portion.

10. The support member according to claim 9, wherein each of the apertures (126) has a diameter, or if not circular a largest dimension across the respective aperture, of less than about 10 µm.

11. The support member according to any one of claims 1 to 10, wherein well-like depression(s) (128) is/are defined in a biological material-contacting surface (130) of the biocompatible support portion (106) of the support member (104).

12. The support member according to any one of Claim s 1 to 11, wherein the biocompatible support portion (106) of the support member (104) comprises a pair of flexible protrusions (132) for supporting tissue thereon and therebetween.

13. The support member according to any one of claims 1 to 12, wherein the support member (104) is mountable to the substrate via insertion of at least part of the support member into at least one chamber unit (110) defined in the substrate (102and wherein the at least one support member is detachable, from the substrate via withdrawal of the at least part of the at least one support member from said at least one chamber unit.

14. The support member according to any one of claims 1 to 13, wherein the biocompatible support portion (106) comprises an elastomeric material that provides flexibility to the biocompatible support portion; optionally wherein the elastomeric material comprises at least one selected from the group consisting of silicone or polybutadiene.

15. The support member according to claim 14, wherein the elastomeric material comprises carboxylic acid groups available at least at the surface of the support portion; and/or wherein the biocompatible support portion (106) comprises a cell culture protein for contacting the biological material (108).

16. A support member (104) for supporting biological material (108), the support member being mountable to, and detachable from, a substrate (102), the support member comprising a biocompatible support portion (106) for supporting the biological material.

17. A substrate for use with a system for testing biological material, wherein the substrate is configured such that a support member of any one of the claims 1 to 16 is mountable to, and detachable from the substrate.

18. The substrate of claim 17, wherein the substrate is configured such that a support member of embodiment 5 is mountable to, and detachable from the substrate, wherein the substrate comprises the second orientation feature.

19. The substrate according to claim 18, wherein the first orientation feature comprises a shape feature (122)) and the second orientation feature comprises a further shape feature (124) and the further shape feature is part of the substrate (102), the shape feature and the further shape feature fitting to each other upon adoption of the defined reference orientation.

20. The substrate of any one of claims 17 to 19, the substrate comprising at least one chamber unit, wherein at least one of the at least one chamber unit is configured to receive at least a part of a support member of any one of the embodiments 1 to 16 such that the biocompatible support portion (106) separates an upper portion of the at least one chamber unit from a lower portion of the at least one chamber unit (110) when the part of the at least one support member (104) is inserted into the at least one chamber unit.

21. A system for testing biological material, the system comprising:
a support member of any of the claims 1 to 16; and
the substrate of any one of the claims 17 to 20.

22. The system (100) according to claim 18, wherein the biocompatible support portion (106) separates an upper portion from a lower portion of the respective chamber unit (110) when the at least part of the at least one support member (104) is inserted into said one of the at least one chamber unit.

23. The substrate or system (100) according to any one of the claims 17 to 22, wherein the at least one chamber unit (110) comprises a plurality of chamber units defined in the substrate (102).

24. The substrate or system (100) according to any one of the claim 17 to 23, wherein the at least one support member (104) comprises a plurality of support members.

25. The substrate or system (100) according to claim 24 as according to any one of claims 13 to 24, wherein two or more support members (104) of the plurality of support members are mountable to the substrate (102) by receiving at least part of each of the two or more support members in said one of the at least one chamber unit (110).

26. Use of the support member, substrate or system (100) according to any one of claims 1 to 25 for testing biological material (108).

27. A method of testing biological material (108), the method comprising:
supporting the biological material on the biocompatible support portion (106) included in the at least one support member (104) of the system (100) according to any one of embodiments 1 to 25,
